# EUROPÄISCHE PATENTSCHRIFT

(11) **EP 3 958 072 B1**
(45) Veröffentlichungstag und Bekanntmachung des Hinweises auf die Patenterteilung: **27.09.2023**
(21) Anmeldenummer: 21000218.4
(22) Anmeldetag: 09.08.2021
(51) Int. Cl.: G05B 19/042, A61M 16/00

(54) **PRIORISIERUNG VON SCHNITTSTELLEN EINES BEATMUNGSGERÄTES**
PRIORITIZATION OF INTERFACES OF A VENTILATOR
HIÉRARCHISATION DES INTERFACES D'UN RESPIRATEUR

(30) Priorität: 20.08.2020 DE 102020121835
(43) Veröffentlichungstag der Anmeldung: 23.02.2022
(73) Patentinhaber: Löwenstein Medical Technology S.A., 2557 Luxembourg (LU)
(72) Erfinder: Schwaibold, Matthias, 76228 Karlsruhe (DE); Skiba, Alexander, 76297 Stutensee (DE); Schröter, Christof, 76307 Karlsbad (DE); Haushammer, Mario, 76133 Karlsruhe (DE)
(74) Vertreter: Marx, Thomas

(56) Entgegenhaltungen:
- WO-A1-2016/128280
- WO-A1-2021/195336

## Beschreibung

Die Erfindung betrifft ein Verfahren zur Verwaltung von Datenaustausch über Schnittstellen eines Beatmungsgerätes.

Moderne Beatmungsgeräte verfügen meist über mehrere Schnittstellen, welche Eingaben und einen Datenaustausch ermöglichen. WO 2016/128280 A1 offenbart ein Beatmungsgerät mit zwei Schnittstellen. Über die verschiedenen Schnittstellen kann ein Beatmungsgerät sowohl direkt am Beatmungsgerät eingestellt werden, aber beispielsweise auch über eine Internetverbindung z.B. per Fernwartung. Bei aktuellen Beatmungsgeräten sind dabei alle Schnittstellen gleichzeitig freigegeben um zum Beispiel Dateneingaben vorzunehmen oder auch gleichzeitig eine Softwareaktualisierung aufzuspielen. Werden gleichzeitig mehrere Dateneingaben über verschiedene Schnittstellen getätigt, kann es vorkommen, dass diese Dateneingaben, z.B. im Sinne der Therapie eines Patienten, nicht zusammenpassen und die Anwendung des Beatmungsgeräts wirkungslos oder gar schädlich werden lässt. Auch tiefergehende Fehlfunktionen des Geräts können nicht ausgeschlossen werden, was ebenfalls zu einer schädlichen Beeinträchtigung des Patienten führen kann. WO 2021/195336 A1 offenbart ein Verfahren zur Verwaltung von Datenaustausch über zumindest drei Schnittstellen eines Beatmungsgerätes, wobei eine Dateneingabe über eine der Schnittstellen die Dateneingabe über zumindest eine andere Schnittstelle sperrt.

Aufgabe der vorliegenden Erfindung ist daher die Bereitstellung eines Verfahrens zur sicheren Steuerung eines Beatmungsgeräts. Die Aufgabe wird durch ein Verfahren nach Anspruch 1 sowie durch die Vorrichtung nach Anspruch 15 gelöst.

Verfahren zur Verwaltung von Datenaustausch über zumindest drei Schnittstellen eines Beatmungsgerätes, wobei zumindest eine Schnittstelle für einen Datenaustausch mit zumindest einer von dem Beatmungsgerät räumlich getrennten Gegenstelle eingerichtet ist, wobei der Datenaustausch eine Dateneingabe und eine Datenausgabe umfasst, wobei eine Dateneingabe über eine der Schnittstellen die Dateneingabe über zumindest eine andere Schnittstelle sperrt. Die Dateneingabe kann darüber hinaus aber auch für mehr als eine andere oder auch alle anderen Schnittstellen gesperrt werden.

In manchen Ausführungsformen des Verfahrens, ist eine Datenausgabe zu jeder Zeit während des laufenden Betriebs des Beatmungsgerätes und bei ausreichenden Systemressourcen und einer sicheren Stromzufuhr über zumindest eine der Schnittstellen möglich. Eine Datenausgabe kann aber durchaus auch über mehrere oder gar alle Schnittstellen möglich sein. Eine sichere Stromzufuhr ist zum Beispiel vorhanden, wenn das Beatmungsgerät an das örtliche Stromnetz angeschlossen ist oder auch ein Akku bzw. Batterie des Beatmungsgerätes ausreichend geladen ist. Eine ausreichende Ladung des Akkus kann beispielsweise bei über 50 % oder 20% der maximalen Ladung vorliegen. Auch eine Ladung von über 10% der maximalen Ladung oder über 5% der maximalen Ladung kann als ausreichende Ladung des Akkus angesehen werden. Auch ist es beispielsweise denkbar, dass das Beatmungsgerät eingerichtet ist, die ausreichende Akkuladung anhand der Art, Dauer und Schnittstelle der Datenausgabe zu bestimmen. So kann für kurze Datenausgaben über Schnittstellen, welche wenig Energie benötigen, eine niedrigere Akkuladung vom Beatmungsgerät als ausreichen angesehen werden. Für längere Datenausgaben und/oder Datenausgaben über Schnittstellen mit höherem Energiebedarf kann eine andere Akkuladung als ausreichend zugrunde gelegt werden. Die Systemressourcen umfassen dabei primär Ressourcen wie zum Beispiel die Prozessorkapazität und Arbeitsspeicher. Beispielsweise kann der Prozessor während des laufenden Betriebs durch die Steuerung des Beatmungsdruckes zu Teilen ausgelastet sein, sodass die Datenausgabe den Prozessor überlasten kann, wodurch wiederum die Sicherheit der Therapie stark gefährdet werden würde. In dem Fall würden beispielsweise die Systemressourcen als nicht ausreichend angesehen. Insbesondere bei der Betrachtung der verfügbaren Systemressourcen kann eine Datenübertragung auch verzögert werden, also nicht sofort ausgeführt, sondern zu einem späteren Zeitpunkt durchgeführt werden. Stehen zu einem Zeitpunkt, zu dem eine Datenausgabe stattfinden soll beispielweise also nicht ausreichend Systemressourcen zur Verfügung, sollte keine Datenausgabe stattfinden. Die Datenausgabe kann dann aber verzögert werden und stattfinden, sobald wieder ausreichend Systemressourcen zur Verfügung stehen.

In manchen Ausführungsformen des Verfahrens ist eine Dateneingabe zu jeder Zeit während des laufenden Betriebs des Beatmungsgerätes und bei ausreichenden Systemressourcen und einer sicheren Stromzufuhr über zumindest eine der Schnittstellen möglich. Eine sichere Stromzufuhr ist zum Beispiel vorhanden, wenn das Beatmungsgerät an das örtliche Stromnetz angeschlossen ist oder auch ein Akku bzw. Batterie des Beatmungsgerätes ausreichend geladen ist. Eine ausreichende Ladung des Akkus kann beispielsweise bei über 50 % oder 20% der maximalen Ladung vorliegen. Auch eine Ladung von über 10% der maximalen Ladung oder über 5% der maximalen Ladung kann als ausreichende Ladung des Akkus angesehen werden. Auch ist es beispielsweise denkbar, dass das Beatmungsgerät eingerichtet ist, die ausreichende Akkuladung anhand der Art, Dauer und Schnittstelle der Dateneingabe zu bestimmen. So kann für kurze Dateneingaben über Schnittstellen, welche wenig Energie benötigen, eine niedrigere Akkuladung vom Beatmungsgerät als ausreichen angesehen werden. Für längere Dateneingaben und/oder Dateneingaben über Schnittstellen mit höherem Energiebedarf kann eine andere Akkuladung als ausreichend zugrunde gelegt werden. Die Systemressourcen umfassen dabei primär Ressourcen wie zum Beispiel die Prozessorkapazität und Arbeitsspeicher. Beispielsweise kann der Prozessor während des laufenden Betriebs durch die Steuerung des Beatmungsdruckes zu Teilen ausgelastet sein, sodass die Dateneingabe den Prozessor überlasten kann, wodurch wiederum die Sicherheit der Therapie stark gefährdet werden würde. In dem Fall würden beispielsweise die Systemressourcen als nicht ausreichend angesehen. Insbesondere bei der Betrachtung der verfügbaren Systemressourcen kann eine Datenübertragung auch verzögert werden, also nicht sofort ausgeführt, sondern zu einem späteren Zeitpunkt durchgeführt werden. Stehen zu einem Zeitpunkt, zu dem eine Dateneingabe stattfinden soll beispielweise also nicht ausreichend Systemressourcen zur Verfügung, sollte keine Dateneingabe stattfinden. Die Dateneingabe kann dann aber verzögert werden und stattfinden, sobald wieder ausreichend Systemressourcen zur Verfügung stehen.

Erfindungsgemäß wird den Schnittstellen eine Priorität zugeordnet und die Dateneingabe über eine Schnittstelle mit höherer Priorität die Dateneingabe über zumindest eine Schnittstelle niedrigerer Priorität sperrt. Die Dateneingabe kann darüber hinaus aber auch für mehr als eine oder auch alle Schnittstellen niedrigerer Priorität gesperrt werden.

In manchen Ausführungsformen des Verfahrens wird die Dateneingabe über zumindest eine Schnittstelle niedrigerer Priorität gesperrt, wenn die Dateneingabe über Schnittstellen niedrigerer Priorität mit der Dateneingabe über die Schnittstelle höherer Priorität in Konflikt steht. Die Dateneingabe kann darüber hinaus aber auch für mehr als eine oder auch alle Schnittstellen niedrigerer Priorität gesperrt werden.

In manchen Ausführungsformen des Verfahrens wird die Dateneingabe zur Änderung der Therapieeinstellungen gegenüber zumindest einer Schnittstelle niedrigerer Priorität gesperrt wird, wenn eine Dateneingabe zur Änderung der Therapieeinstellungen über eine Schnittstelle höherer Priorität erfolgt. Die Dateneingabe zur Änderung der Therapieeinstellungen kann darüber hinaus aber auch für mehr als eine oder auch alle Schnittstellen niedrigerer Priorität gesperrt werden.

In manchen Ausführungsformen des Verfahrens umfasst das Beatmungsgerät zumindest drei verschiedene Schnittstellen, wobei durch diese zumindest drei Schnittstellen zumindest drei der Gruppen a bis e repräsentiert werden:
a. Drahtlose Schnittstellen für große Entfernungen
b. Drahtlose Schnittstellen für kleine Entfernungen
c. Benutzerschnittstelle
d. Kabelgebundene Schnittstellen
e. Schnittstellen für ein Speichermedium.

In manchen Ausführungsformen des Verfahrens umfasst das Beatmungsgerät zumindest fünf verschiedene Schnittstellen, wobei das Beatmungsgerät zumindest eine Schnittstelle aus jeder der Gruppen a bis e aufweist:
a. Drahtlose Schnittstellen für große Entfernungen
b. Drahtlose Schnittstellen für kleine Entfernungen
c. Benutzerschnittstelle
d. Kabelgebundene Schnittstellen
e. Schnittstellen für ein Speichermedium.

In manchen Ausführungsformen des Verfahrens umfasst das Beatmungsgerät zumindest drei verschiedene Schnittstellen, wobei durch diese zumindest drei Schnittstellen zumindest drei der Gruppen a bis f repräsentiert werden:
a. Drahtlose Schnittstellen für große Entfernungen
b. Drahtlose Schnittstellen für kleine Entfernungen
c. Benutzerschnittstelle
d. Kabelgebundene Schnittstellen
e. Schnittstellen für ein Speichermedium
f. Patienten-Interface.

In manchen Ausführungsformen des Verfahrens umfasst das Beatmungsgerät zumindest sechs verschiedene Schnittstellen, wobei das Beatmungsgerät zumindest eine Schnittstelle aus jeder der Gruppen a bis f aufweist:
a. Drahtlose Schnittstellen für große Entfernungen
b. Drahtlose Schnittstellen für kleine Entfernungen
c. Benutzerschnittstelle
d. Kabelgebundene Schnittstellen
e. Schnittstellen für ein Speichermedium
f. Patienten-Interface.

In manchen Ausführungsformen des Verfahrens können die drahtlosen Schnittstellen des Beatmungsgerätes aktiviert und deaktiviert werden.

In manchen Ausführungsformen des Verfahrens ist eine der Schnittstellen eine Benutzerschnittstelle am Beatmungsgerät und der Benutzerschnittstelle wird die höchste Priorität bezüglich der Dateneingabe zugeordnet.

In manchen Ausführungsformen des Verfahrens werden im Wesentlichen alle Dateneingaben über Schnittstellen in das Therapiegerät während des laufenden Betriebs des Beatmungsgeräts gesperrt.

In manchen Ausführungsformen des Verfahrens werden therapiebezogene Dateneingaben während des laufenden Betriebs des Beatmungsgerätes gesperrt.

In manchen Ausführungsformen des Verfahrens kann über zumindest eine Schnittstelle die Prioritätszuordnung umgangen werden, sodass Dateneingaben über diese Schnittstelle den Dateneingaben auch über Schnittstellen der höchsten Priorität vorgezogen werden. Diese Möglichkeit kann auch auf mehrere oder alle Schnittstellen ausgeweitet werden.

In manchen Ausführungsformen des Verfahrens wird die Priorität der Schnittstellen anhand der Entfernung zwischen dem Beatmungsgerät und der Gegenstelle festgelegt.

In manchen Ausführungsformen des Verfahrens wird zur Dateneingabe ein Dateneingabemodus gestartet und das Starten des Dateneingabemodus über eine Schnittstelle das Starten eines Dateneingabemodus über die anderen Schnittstellen sperrt.

In manchen Ausführungsformen des Verfahrens werden für verschiedene Dateneingaben jeweils verschiedene Dateneingabemodi gestartet, wobei das Starten eines Dateneingabemodus über eine Schnittstelle das Starten des gleichen Dateneingabemodus durch eine andere Schnittstelle niedrigerer Priorität sperrt.

In manchen Ausführungsformen des Verfahrens wird das Starten eines Dateneingabemodus für eine Dateneingabe über andere Schnittstellen gesperrt, wenn der zu startende Dateneingabemodus über Schnittstellen niedrigerer Priorität mit dem Dateneingabemodus der Dateneingabe über Schnittstellen höherer Priorität im Konflikt steht.

In manchen Ausführungsformen des Verfahrens wird die Dateneingabe über eine Schnittstelle durch den Beginn einer Dateneingabe durch eine Schnittstelle, der eine höhere Priorität zugeordnet ist, unterbrochen und/oder abgebrochen.

In manchen Ausführungsformen des Verfahrens sperrt die Aktivierung von Funktionen und/oder Arbeitsweisen des Beatmungsgeräts den Datenaustausch über zumindest eine Schnittstelle. Die Aktivierung von Funktionen und/oder Arbeitsweisen kann in alternativen Ausführungsformen auch dazu führen, dass mehrere oder auch alle Schnittstellen gesperrt werden.

In manchen Ausführungsformen des Verfahrens deaktiviert die Aktivierung von Funktionen und/oder Arbeitsweisen des Beatmungsgeräts zumindest eine Schnittstelle bis zur Beendigung der Funktion und/oder Arbeitsweise. Die Aktivierung von Funktionen und/oder Arbeitsweisen kann in alternativen Ausführungsformen auch dazu führen, dass mehrere oder auch alle Schnittstellen deaktiviert werden.

In manchen Ausführungsformen des Verfahrens müssen bestimmte Dateneingaben über eine Schnittstelle über eine Schnittstelle höherer Priorität bestätigt werden. Diese bestimmten Aktionen und/oder Dateneingaben können beispielsweise kritische und/oder sensible Aspekte des Patienten und/oder der Therapie betreffen. Beispielsweise ist darunter auch ein Umschalten des Beatmungsgerätes vom nicht-laufenden Betrieb in den laufenden Betrieb oder umgekehrt zu verstehen. Auch Änderungen der Therapieeinstellungen müssen gegebenenfalls über eine Schnittstelle höherer Priorität bestätigt werden. Es kann alternativ oder ergänzend vorgesehen sein, dass Geräteaktualisierungen, also Software und/oder Firmware-Updates, ebenfalls über eine Schnittstelle höherer Priorität bestätigt werden müssen.

In manchen Ausführungsformen des Verfahrens werden bestimmte Dateneingaben unabhängig von der Priorität der Schnittstelle über welche die Dateneingabe erfolgt nicht unterbrochen und/oder abgebrochen.

In manchen Ausführungsformen des Verfahrens wird durch Dateneingaben und/oder Datenausgaben über eine Schnittstelle eine Meldung generiert, welche über zumindest eine Schnittstelle höherer Priorität ausgegeben wird.

In manchen Ausführungsformen des Verfahrens bleibt die Meldung über die Dauer der Dateneingabe bestehen.

In manchen Ausführungsformen des Verfahrens bleibt die Meldung nach der Dateneingabe bestehen und muss optional über die Schnittstelle, über welche die Meldung ausgegeben wird, bestätigt werden.

In manchen Ausführungsformen des Verfahrens wird nach der Dateneingabe über eine Schnittstelle eine Meldung generiert und über zumindest eine Schnittstelle höherer Priorität ausgegeben, wobei die Meldung eine Zusammenfassung über die erfolgte Dateneingabe enthält.

In manchen Ausführungsformen des Verfahrens basiert ein Kriterium, nach dem die Priorität den Schnittstellen zugeordnet wird, auf der Verbindung der Schnittstelle zu der räumlich getrennten Gegenstelle.

In manchen Ausführungsformen des Verfahrens wird den Schnittstellen die Priorität automatisch zugeordnet.

In manchen Ausführungsformen des Verfahrens ist die Priorität der Schnittstellen am Beatmungsgerät manuell veränderbar.

In manchen Ausführungsformen des Verfahrens sind die Bestandteile und Komponenten des Beatmungsgeräts in Module und/oder Modulgruppen eingeteilt, wobei die Dateneingabe von über eine Schnittstelle nur das betroffene Modul und/oder die betroffene Modulgruppe gegenüber Dateneingaben über andere Schnittstellen sperrt.

In manchen Ausführungsformen des Verfahrens wird die Priorität der Schnittstellen zumindest für den laufenden Betrieb und den nicht-laufenden Betrieb unterschiedlich vergeben.

In manchen Ausführungsformen des Verfahrens ist eine der Schnittstellen als USB-Schnittstelle ausgeführt und bei einer aktiven Verbindung über diese USB-Schnittstelle geht das Beatmungsgerät nicht in den laufenden Betrieb über.

In manchen Ausführungsformen des Verfahrens ist während des laufenden Betriebs die USB-Schnittstelle des Beatmungsgerätes deaktiviert.

In manchen Ausführungsformen des Verfahrens geht das Beatmungsgerät vom laufenden Betrieb in den nicht-laufenden Betrieb über, sobald eine Verbindung über die USB-Schnittstelle hergestellt wird.

In manchen Ausführungsformen des Verfahrens wird bei der Herstellung einer Verbindung über die USB-Schnittstelle eine Dateneingabe über alle anderen Schnittstellen gesperrt.

In manchen Ausführungsformen des Verfahrens werden alle Schnittstellen gegenüber therapiebezogenen Eingaben am Beatmungsgerät zumindest zeitweise gesperrt, nachdem eine therapiebezogene Eingabe über eine Schnittstelle abgeschlossen wurde.

In manchen Ausführungsformen des Verfahrens wird eine Änderung des Beatmungsgeräts vom laufenden in den nicht-laufenden Betrieb oder umgekehrt zumindest zeitweise über alle Schnittstellen gesperrt, nachdem eine solche Änderung vorgenommen wurde.

In manchen Ausführungsformen des Verfahrens wird eine Dateneingabe über alle Schnittstellen, insbesondere über die Benutzerschnittstelle, gesperrt, während das Beatmungsgerät während des laufenden Betriebs aufgezeichnete Daten verarbeitet.

In manchen Ausführungsformen des Verfahrens wird die Dateneingabe über alle Schnittstellen im Wesentlichen gesperrt, wenn das Beatmungsgerät und/oder die Firmware des Beatmungsgerätes einen schwerwiegenden Fehler registriert. Insbesondere technische Fehler können als solche schwerwiegenden Fehler betrachtet werden. Dabei ist zum Beispiel an defekte Sensoren oder defekte Elektronik zu denken.

In manchen Ausführungsformen des Verfahrens werden im Wesentlichen alle drahtlosen Verbindungen über entsprechende Schnittstellen des Beatmungsgerätes abgebrochen oder unterbrochen und die Schnittstellen für drahtlose Verbindungen deaktiviert, wenn das Beatmugsgerät in den laufenden Betrieb übergeht und/oder übergehen soll.

In manchen Ausführungsformen des Verfahrens geht das Beatmungsgerät nicht in den laufenden Betrieb über bis alle drahtlosen Verbindungen unterbrochen und die Schnittstellen für drahtlose Verbindungen deaktiviert sind.

In manchen Ausführungsformen des Verfahrens sind bestimmte Aktionen und/oder Dateneingaben über die Benutzerschnittstelle unabhängig von der Priorisierung immer möglich.

Das Beatmungsgerät umfasst zumindest drei Schnittstellen für einen Datenaustausch, wobei zumindest eine Schnittstelle für einen Datenaustausch mit zumindest einer von dem Beatmungsgerät räumlich getrennten Gegenstelle eingerichtet ist, wobei der Datenaustausch eine Dateneingabe und eine Datenausgabe umfasst, wobei eine Dateneingabe über die Schnittstelle die Dateneingabe über zumindest eine andere Schnittstelle sperrt. Die Dateneingabe kann darüber hinaus aber auch für mehr als eine andere oder auch alle anderen Schnittstellen gesperrt werden.

In manchen Ausführungsformen des Beatmungsgerätes ist eine Datenausgabe zu jeder Zeit während des laufenden Betriebs des Beatmungsgerätes und bei ausreichenden Systemressourcen und einer sicheren Stromzufuhr über zumindest eine der Schnittstellen möglich ist.

Erfindungsgemäß wird den Schnittstellen eine Priorität zugeordnet und die Dateneingabe über eine Schnittstelle mit höherer Priorität sperrt die Dateneingabe über zumindest eine Schnittstelle niedrigerer Priorität sperrt. Die Dateneingabe kann darüber hinaus aber auch für mehr als eine oder auch alle Schnittstellen niedrigerer Priorität gesperrt werden.

In manchen Ausführungsformen des Beatmungsgerätes umfasst das Beatmungsgerät zumindest drei verschiedene Schnittstellen, wobei durch diese zumindest drei Schnittstellen zumindest drei der Gruppen a bis e repräsentiert werden:
a. Drahtlose Schnittstellen für große Entfernungen
b. Drahtlose Schnittstellen für kleine Entfernungen
c. Benutzerschnittstelle
d. Kabelgebundene Schnittstellen
e. Schnittstellen für ein Speichermedium.

Es ist darauf hinzuweisen, dass die in den Ansprüchen einzeln aufgeführten Merkmale in beliebiger, technisch sinnvoller Weise miteinander kombiniert werden können und weitere Ausgestaltungen der Erfindung aufzeigen. Die Beschreibung charakterisiert und spezifiziert die Erfindung insbesondere im Zusammenhang mit den Figuren zusätzlich.

Es sei ferner darauf hingewiesen, dass eine hierin verwendete, zwischen zwei Merkmalen stehende und diese miteinander verknüpfende Konjunktion "und/oder" stets so auszulegen ist, dass in einer ersten Ausgestaltung des erfindungsgemäßen Gegenstands lediglich das erste Merkmal vorhanden sein kann, in einer zweiten Ausgestaltung lediglich das zweite Merkmal vorhanden sein kann und in einer dritten Ausgestaltung sowohl das erste als auch das zweite Merkmal vorhanden sein können.

Unter einem Beatmungsgerät ist jedwedes Gerät zu verstehen, welches einen Anwender oder Patienten bei der natürlichen Atmung unterstützt, die Beatmung des Anwenders bzw. Patienten übernimmt und/oder zur Atemtherapie dient und/oder anderweitig die Atmung des Anwenders bzw. Patienten beeinflusst. Darunter fallen zum Beispiel, aber nicht ausschließend, CPAPsowie BiPAP-Geräte, Narkose- bzw. Anästhesiegeräte, Atemtherapiegeräte, (klinische, außerklinische oder Notfall-) Beatmungsgeräte, Highflow-Therapiegeräte und Hustenmaschinen. Beatmungsgeräte können auch als Diagnosegeräte für die Beatmung verstanden werden. Diagnosegeräte können dabei allgemein zur Erfassung von medizinischen Parametern eines Patienten dienen. Darunter fallen auch Geräte, welche medizinische Parameter von Patienten in Kombination mit oder ausschließlich die Atmung betreffend, erfassen und optional verarbeiten können.

Eine Schnittstelle ist im weitesten Sinne als jede Art von Einrichtung zur Verbindung - unabhängig der Verbindungsart - zwischen dem Beatmungsgerät und einer Gegenstelle oder einer Person zu verstehen. Eine Schnittstelle für eine Verbindung zwischen dem Beatmungsgerät und einer Person kann dabei zum Beispiel eine Benutzerschnittstelle sein, welche die Interaktion zwischen Person und Beatmungsgerät direkt am Beatmungsgerät ermöglicht.

Als Patienten-Interface kann, soweit nicht ausdrücklich anders beschrieben, jeglicher Teil oder verbundene Peripheriegeräte des Beatmungsgerätes verstanden werden, welche zur Interaktion, insbesondere zu Therapie- oder Diagnosezwecken, mit einem Patienten gedacht ist. Insbesondere kann ein Patienten-Interface als Maske eines Beatmungsgerätes bzw. eine mit dem Beatmungsgerät verbundene Maske verstanden werden. Diese Maske kann eine Full-Face, also Nase und Mund umschließende, Maske oder eine Nasenmaske, also eine nur die Nase umschließende Maske, sein. Auch Trachealtuben und sogenannte Nasenbrillen können als Maske angesehen werden.

Die einzelnen Bestandteile des Beatmungsgeräts können in Module unterteilt sein, wobei auch das gesamte Beatmungsgerät als ein einziges Modul verstanden werden kann. Die Definition eines Moduls kann unter verschiedenen Gesichtspunkten erfolgen, welche an dieser Stelle nicht näher erläutert werden und keinen Einfluss auf die Erfindung als solche haben. Auch ein Überschneiden von verschiedenen Modulen durch die Bestandteile des Beatmungsgeräts ist dabei möglich. So kann ein Bestandteil beispielsweise zu zwei Modulen zugeordnet sein. Weiter können die einzelnen Module auch zu Modulgruppen zusammengefasst werde, wobei auch diese Modulgruppen unter Umständen Überschneidungen aufweisen können.

Die Quelle einer Dateneingabe kann verschiedene Formen haben, resultiert aber in der Regel zumindest indirekt in einem elektrischen Signal, welches innerhalb des Beatmungsgeräts verarbeitet werden kann. Beispielsweise kann die Dateneingabe mechanisch über eine Benutzerschnittstelle in Form eines berührungssensitiven Bildschirms erfolgen. Über den berührungssensitiven Bildschirm wird die mechanische Berührung in ein elektrisches Signal umgewandelt, welches im Beatmungsgerät verarbeitet werden kann. Über andere Schnittstellen kann beispielsweise aber auch direkt ein elektrisches Signal eingegeben werden. An dieser Stelle wird von einer genauen Beischreibung aller möglichen Wege einer Dateneingabe am Beatmungsgerät zu tätigen bzw. über eine Schnittstelle zu übermitteln verzichtet, da die Form der Dateneingabe keine Relevanz für das erfinderische Verfahren bzw. die erfinderische Vorrichtung hat.

Im Wesentlichen kann bei dem Beatmungsgerät zwischen einem laufenden Betrieb und einem nicht-laufenden Betrieb unterschieden werden. Ein laufender Betrieb meint dabei, soweit nicht explizit anders angegeben, die Benutzung des Beatmungsgeräts an beispielsweise einem Patienten. Im Fall von Therapiegeräten kann dies beispielsweise eine aktive Therapie sein, welche am Patienten durchgeführt wird. Ist das Beatmungsgerät beispielsweise als Atemtherapiegerät ausgelegt, so befindet sich das Atemtherapiegerät im laufenden Betrieb, wenn der Patient an das Atemtherapiegerät angeschlossen ist und ein Therapieprogramm läuft, der Patient also beatmet wird beziehungsweise bei der Atmung unterstützt wird. Ist das Beatmungsgerät beispielsweise ein Diagnosegerät, dann ist als laufender Betrieb die Verwendung des Diagnosegerätes um eine Diagnose an zum Beispiel einem Patienten durchzuführen beziehungsweise die Aufnahme von Daten zur Diagnose. Das Beatmungsgerät ist im nicht-laufenden Betrieb, wenn das Beatmungsgerät nicht aktiv genutzt wird. Dies kann also beispielsweise sein, wenn sich das Beatmungsgerät in einem Stand-By Modus, einem sogenannten "Sleep"-Modus oder auch im ausgeschalteten Zustand - unabhängig davon ob eine Stromverbindung besteht oder nicht - befindet.

Ein von dem Beatmungsgerät räumlich getrennte Gegenstelle beschreibt, soweit nicht explizit anders angegeben, eine Gegenstelle, welche nicht zwingend dauerhaft mit dem Beatmungsgerät elektrisch und/oder mechanisch verbunden ist und auch nicht für den Betrieb essentiell mit dem Beatmungsgerät dauerhaft verbunden sein muss. Beispielsweise kann unter einer räumlich getrennten Gegenstelle auch kabelgebundene Geräte oder auch Speichermedien, wie zum Beispiel SD-Karten, USB-Sticks, etc., verstanden werden, obwohl diese zumindest zeitweise unmittelbar räumlich mit dem Beatmungsgerät verbunden werden können bzw. sind

Die Priorität der Schnittstellen kann anhand verschiedenster Aspekte bestimmt und eingestellt werden. Eine Möglichkeit der Prioritätsvergabe kann zum Beispiel manuell erfolgen, sodass der Anwender die Priorität der Schnittstellen ganz frei vergeben kann. Insbesondere die Benutzerschnittstelle, welche unter anderem eine Dateneingabe direkt am Beatmungsgerät ermöglicht, kann dauerhaft und optional unveränderlich die höchste Priorität zugeordnet sein. Neben der Vergabe der höchsten Priorität an die Benutzerschnittstelle ist auch die Vergabe der höchsten Priorität an bestimmte Schnittstellen, wie zum Beispiel Service-Schnittstellen möglich. Teilen sich Service-Gegenstellen und/oder Gegenstellen eines Prüfstandes die Schnittstelle mit anderen Gegenstellen, so ist auch eine Gegenstellen-abhängige Priorität für diese Schnittstellen denkbar. Beispielsweise können diese Schnittstellen auch doppelt in der Prioritätszuordnung geführt werden, sodass ein extra Merkmal zur Prioritätszuordnung vermerkt ist. Eine weitere Möglichkeit etwaigen Gegenstellen eines Prüfstandes oder Service-Gegenstellen die Dateneingabe unter höchster Priorität - also auch über der Benutzerschnittstelle - zu ermöglichen, kann in Form einer Umgehung oder eines Überschreibens beziehungsweise Überbrückens der Priorität durch die Gegenstellen implementiert werden. Dazu wäre beispielsweise auch die Übermittlung eines Überbrückungsbefehls durch die jeweilige Gegenstelle möglich, sodass das Beatmungsgerät erkennt, dass die Prioritätsreihenfolge außer Kraft gesetzt werden soll und/oder der sendenden Gegenstelle vorrübergehend eine erhöhte (die höchste) Priorität zugeordnet werden soll. Eine Festlegung der Prioritäten kann neben der manuellen Vergabe auch automatisch erfolgen. Dabei können verschiedene Kriterien festgelegt werden, wonach die Priorität vergeben wird.

Auch ist beispielsweise eine unterschiedliche Prioritätsvergabe möglich, je nachdem ob sich das Beatmungsgerät im laufenden oder im nicht-laufenden Betrieb befindet. Eine andere Möglichkeit der Unterscheidung zwischen laufendem Betrieb und nicht-laufendem Betrieb kann beispielsweise so eingerichtet werden, dass dem laufenden Betrieb grundsätzlich die höchste Priorität zugeordnet wird. Der laufende Betrieb kann in einigen Ausführungsformen daher auch nicht oder nur unter bestimmten Voraussetzungen - wie zum Beispiel einer Fehlfunktion des Beatmungsgerätes - abgebrochen werden. Solche Fehlfunktionen können beispielsweise in der eigentlichen Funktionsweise liegen aber auch durch fehlerhafte Peripheriegeräte und/oder Sensoren hervorgerufen werden. Bleibt während des laufenden Betriebs beispielsweise die Dateneingabe durch Sensoren des Beatmungsgerätes oder mit dem Beatmungsgerät verbundene Sensoren aus, so kann das Beatmungsgerät unter anderem von einer Fehlfunktion ausgehen und daraufhin den laufenden Betrieb beenden.

Beispielsweise kann die Priorität anhand einer Entfernung zwischen Beatmungsgerät und Gegenstelle festgelegt werden. Die reine physikalische Entfernung ist hierbei ein untergeordnetes beziehungsweise zu vernachlässigendes Kriterium, kann aber durchaus in weiteren Ausführungsformen als Kriterium für die Priorität herangezogen werden. Beispielsweise kann die maximale Entfernung zwischen Beatmungsgerät und Gegenstelle als Hauptkriterium für die Zuordnung der Priorität zu Schnittstellen herangezogen werden. Folglich würde unter diesem Gesichtspunkt einer Benutzerschnittstelle direkt am Beatmungsgerät die höchste Priorität zugeordnet. Schnittstellen für kurze Entfernungen, zum Beispiel für drahtlose Verbindungen wie Bluetooth oder andere WPAN-Verbindungen (Wireless Personal Area Network) oder auch Kurzstreckenfunkverbindungen, können nach diesem Kriterium ebenfalls höhere Prioritäten bezüglich der Dateneingabe zugeordnet werden. Die nächst tiefere Stufe der Prioritäten kann beispielsweise dann den Schnittstellen für Verbindungen in dem Gebäude, in dem das Beatmungsgerät verwendet wird, zugeordnet werden. Darunter kann beispielsweise eine Fernsteuerung in einem Schlaflabor, aber auch zum Beispiel eine zentrale Station in einer Klinik zählen. Weitere dieser Art von Schnittstellen können beispielsweise zum Auslesen detaillierter Therapiedaten, einer Verbindung zu Diagnose-Geräten (PSG), zum externen Anschluss eines Funkmoduls, allgemeinem Service, der Kommunikation mit einem Prüfstand oder Firmware-Aktualisierungen dienen. Nach der beispielhaft ausgeführten Prioritätsvergabe werden Schnittstellen für Verbindungen über große Entfernungen, wie z.B. Verbindungen mit dem Internet oder dem Mobilfunk, geringe Prioritäten zugeordnet. Für Schnittstellen für Speichermedien wie Speicherkarten oder USB-Sticks kann beispielsweise eine Ausnahmeregelung getroffen werden, nach der beispielsweise die geringste Priorität an ebenjene Schnittstellen für Speichermedien vergeben wird. Darüber hinaus ist aber auch die Einordnung mit einer hohen Priorität, in manchen Fällen auch höher als die Priorität der Benutzerschnittstelle, denkbar.

Eine andere Methode oder Kriterium zur Festlegung der Priorität von Schnittstellen kann beispielsweise auch die Stabilität oder Qualität der Verbindung der Schnittstelle mit einbeziehen. Beispielsweise kann so die Priorität von verschiedenen Schnittstellen der gleichen Art, wie zum Beispiel Schnittstellen zur Verbindung über große Entfernungen (z.B. über Kabel/LAN, Wifi/WLAN, Mobilfunk zum Internet) weiter differenziert werden. Ist das Beatmungsgerät beispielsweise über eine Schnittstelle per Kabel stabil und mit hoher Übertragungsqualität mit dem Internet verbunden und es besteht gleichzeitig über eine andere Schnittstelle eine Verbindung per Mobilfunk, aber eher instabil oder mit geringer Übertragungsqualität, kann der Mobilfunk-Schnittstelle eine geringere Priorität als der Kabel-Schnittstelle zugeordnet werden.

Bei einer beispielhaften Ausführungsform der Erfindung wird daher die Priorität der drahtlosen Schnittstellen anhand der Verbindungsqualität automatisch bestimmt. Hat beispielsweise eine Schnittstelle eine drahtlose Verbindung mit dem Mobilfunk-Internet, deren Qualität (zum Beispiel in Form der Übertragungsgeschwindigkeit oder Verbindungsstabilität) schlecht ist und hat gleichzeitig eine andere Schnittstelle- eine Verbindung mit dem Internet über WLAN, deren Übertragungsgeschwindigkeit und Stabilität besser als die Mobilfunkverbindung ist, kann der Schnittstelle mit Verbindung über WLAN automatisch oder auch manuell eine höhere Priorität zugeordnet werden. Gleiches kann darüber auch zusätzlich für kabelgebundene Verbindungen gelten. Ist beispielsweise ein externes Steuergerät über ein Kabel mit einer Schnittstelle verbunden, kann dieser Verbindung eine höhere Priorität bezüglich Eingaben zugeordnet werden. Zusätzlich zu der Priorität ist es auch denkbar, dass Schnittstellen, welche das gleiche Verbindungsziel - z.B. das Internet - haben, so geschaltet werden können (automatisch oder manuell), dass eine Verbindung nur über jeweils eine der Schnittstellen hergestellt wird. So kann beispielsweise in einem Beatmungsgerät, welches eine Mobilfunk-Schnittstelle, eine WLAN/Wifi-Schnittstelle sowie eine kabelgebundene Schnittstelle für LAN/Internetverbindungen aufweist, nur die jeweils beste Qualität aktiviert werden. Ist beispielsweise die Wifi-Verbindung schlecht oder sehr anfällig für Störung und eine Kabelverbindung nicht vorhanden, kann die Verbindung über die Mobilfunk-Schnittstelle bevorzugt werden und als einzige aktiv geschalten werden.

Die Priorität kann beispielsweise in einer Rangliste dargestellt werden, bei denen die Schnittstellen entsprechend der vorgesehenen Priorität geordnet werden. Die Priorisierung der Schnittstellen kann in manchen Ausführungsformen auch einem Punktesystem folgen. So kann jeder Schnittstelle basierend auf verschiedensten Kriterien und optional auch einer Wertung der jeweiligen Kriterien eine Punktzahl errechnet werden. Beispielsweise kann eine höhere Punktzahl eine höhere Priorität zur Folge haben. Neben fest vergebenen Prioritäten ist auch eine dynamische Vergabe der Prioritäten für die Schnittstellen denkbar. In manchen Ausführungsformen ist zudem auch die Möglichkeit vorgesehen, dass die Priorität über die Benutzerschnittstelle oder auch andere Schnittstellen im gewissen Maße oder völlig frei angepasst werden kann.

In manchen beispielhaften Ausführungsformen kann einer Schnittstelle für Kabelverbindungen, wie zum Beispiel einer USB-Schnittstelle, die höchste Priorität zugeordnet werden, sodass diese Priorität noch über dem laufenden Betrieb steht. So kann ein Verbindungsaufbau über eine USB-Schnittstelle durchaus dazu führen, dass der laufende Betrieb unter- oder abgebrochen bzw. beendet wird. In manchen Ausführungsformen kann die Priorität der Schnittstellen auch entsprechend dynamisch angepasst werden, wobei die Priorität dann auf der mit der Schnittstelle verbundenen Gegenstelle basiert. Wird beispielsweise eine leere SD-Speicherkarte mit einer entsprechend ausgebildeten Schnittstelle verbunden, so kann dies zu einer niedrigeren Priorität führen, als wenn zum Beispiel eine SD-Speicherkarte mit einer Master-Konfiguration mit der Schnittstelle verbunden wird. Ist eine derartige Prioritätsvergabe vorgesehen, so wird zum Beispiel der Inhalt der SD-Karte oder des USB-Sticks zunächst gescannt ohne eine konkrete Dateneingabe vorzunehmen. Wird dabei festgestellt, dass die Priorität des USB-Sticks oder der SD-Karte eine, zumindest vorrübergehend, sehr hohe oder die höchste Priorität zugeordnet werden soll - etwa in Form einer entsprechenden, auf dem USB-Stick oder der SD-Karte enthaltenen Anweisung - dann wird der entsprechende Schnittstelle zunächst die hohe Priorität zugeordnet, was auch eine hohe Priorität bei der Dateneingabe über die entsprechende Schnittstelle zur Folge hat.

In manchen Ausführungsformen wird die Dateneingabe über andere Schnittstellen gesperrt, unabhängig davon, ob eine Schnittstelle, über welche eine Dateneingabe begonnen werden soll, eine höhere Priorität zugeordnet ist. Dies kann durchaus auch zum Beispiel nur bestimmte Aktionen oder Dateneingaben betreffen. Beispielsweise kann eine Konfiguration des Beatmungsgerätes über ein Speichermedium dazu führen, dass eine Dateneingabe im Sinne einer Konfiguration über andere Schnittstellen - beispielsweise einer Netzwerkverbindung - gesperrt wird.

Wird ganz allgemein eine Dateneingabe über eine der Schnittstellen des Beatmungsgeräts vorgenommen, so wird die Dateneingabe über andere Schnittstellen gesperrt, sodass Dateneingaben immer nur über eine Schnittstelle möglich sind. Beispielsweise kann das Beatmungsgerät alternativ auch so eingerichtet sein, dass bei einer Dateneingabe über eine Schnittstelle bestimmte Dateneingaben über andere Schnittstellen weiterhin möglich sind. Beispielsweise können lediglich die von der Dateneingabe betroffenen Bestandteile des Beatmungsgeräts gegenüber Dateneingaben über andere Schnittstellen gesperrt werden.

Bei manchen Dateneingaben ist zudem zum Beispiel ein Abwarten auf die Umstellung des laufenden Betriebs auf den nicht-laufenden Betrieb des Beatmungsgerätes vorgesehen. Soll beispielsweise eine Dateneingabe im Sinne einer Konfigurationsänderung erfolgen, so kann diese nicht vorgenommen werden bis der laufende Betrieb beendet ist. Beispielsweise kann während des laufenden Betriebs ein Speichermedium mit einer neuen Konfiguration mit dem Beatmungsgerät über eine Schnittstelle verbunden werden, diese Konfiguration wird aber frühestens nach Beendigung des laufenden Betriebs auf das Beatmungsgerät übertragen werden beziehungsweise installiert werden. Andererseits kann auch eine Umstellung in den laufenden Betrieb verhindert werden, solange Dateneingaben über Schnittstellen, wie beispielsweise das Übertragen einer Konfiguration, erfolgen. Verfügt das Beatmungsgerät über eine Art automatische Start-Funktion, welche das Beatmungsgerät in den laufenden Betrieb umstellt, wenn eine Benutzung des Beatmungsgeräts begonnen werden soll bzw. begonnen wird, kann auch diese Funktion während Dateneingaben blockiert werden. Eine solche automatische Start-Funktion kann beispielsweise durch das Anlegen einer Peripherieeinrichtung am Patienten oder - am Beispiel eines Beatmungsgerätes - der Beginn der Atmung des Patienten in das Gerät beziehungsweise mit dem Gerät über eine Patientenschnittstelle (Maske) ausgelöst werden. In alternativen Ausführungsformen kann auch andersherum jegliche Dateneingabe beendet oder abgebrochen werden und auch zumindest einige Schnittstellen gesperrt werden, sollte das Beatmungsgerät über die automatische Start-Funktion in den laufenden Betrieb übergehen. Atmet also zum Beispiel der Patient in die Maske ein bzw. benutzt die Maske des Beatmungsgerätes, so unterbricht das Gerät zumindest einige Dateneingaben und/oder Übertragungen und sperrt zumindest manche Schnittstellen - insbesondere drahtlose Schnittstellen für große Entfernungen - für Dateneingaben. In manchen Ausführungsformen kann daher eine Patientenschnittstelle - z.B. in Form einer Maske (Beatmungsmaske, aber auch Arten von Trachealtuben oder Nasenbrillen) - auch zu den Schnittstellen des Beatmungsgeräts zählen. In dem Fall können Patientenschnittstellen eine weitere Gruppe f von Schnittstellen darstellen, welche zumindest einmal repräsentiert sein müssen oder aus denen zumindest ausgewählt wird.

Um Dateneingaben über andere Schnittstellen zu sperren, während eine Dateneingabe bereits über eine Schnittstelle erfolgt, gibt es mehrere denkbare Möglichkeiten. Eine der Möglichkeiten kann beispielsweise darin bestehen, dass die, für diese Eingabe nicht verwendeten, Schnittstellen für Eingaben von Dateneingaben generell gesperrt werden. So kann es beispielsweise eingerichtet sein, dass den entfernten Gegenstellen erst gar nicht ermöglicht wird, die Schnittstelle zu kontaktieren oder auch sämtliche Eingaben über die Schnittstelle werden nicht weitergegeben, sondern unmittelbar nach Erhalt/Eintreffen in der Schnittstelle verworfen. Eine weitere Möglichkeit besteht darin, dass nicht grundsätzlich alle Schnittstellen bezüglich einer Dateneingabe blockiert werden, sondern leidglich die von der Dateneingabe betroffenen Komponenten.

Weiter kann beispielsweise auch eine Steuereinheit dazu ausgebildet sein, die Dateneingabe über Schnittstellen zu sperren beziehungsweise zu ermöglichen. So kann unter gegebenen Umständen eine Dateneingabe in Richtung Beatmungsgerät immer möglich sein, es wird jedoch durch die Steuereinheit anhand der Priorität entschieden ob diese Dateneingabe übernommen, weitergeleitet und/oder gespeichert wird. Für drahtlose Schnittstellen für große Entfernungen kann ein solcher Umstand zum Beispiel nicht gegeben sein, wenn sich das Beatmungsgerät im laufenden Betrieb befindet und deshalb ebenjene Schnittstellen deaktiviert sind, also gar nicht erreichbar für Dateneingaben sind.

Die Priorität der Schnittstellen spielt insbesondere dann eine Rolle, wenn Dateneingaben am Beatmungsgerät allgemein vorgenommen werden. Wird eine Dateneingabe über eine Schnittstelle einer mittleren Priorität vorgenommen, so ist zumindest die Komponente, bei welchem die Dateneingabe vorgenommen wird, gegenüber Veränderungen/Dateneingaben über zumindest einer Schnittstelle niedrigerer Priorität gesperrt. Es kann aber auch gegenüber mehr als einer oder auch allen Schnittstellen niedrigerer Priorität gesperrt werden. Eine Dateneingabe ist allgemein beispielsweise nur dann möglich, wenn keine Dateneingabe über eine andere Schnittstelle erfolgt. Es kann beispielsweise auch möglich sein, dass das Beatmungsgerät bei einer Dateneingabe lediglich gegenüber Schnittstellen gesperrt wird, die eine niedrigere Priorität besitzen als die Schnittstelle, über welche die derzeitige Dateneingabe vorgenommen wird. Soll zum Beispiel eine Dateneingabe über eine Schnittstelle höherer Priorität erfolgen, so wird die aktuelle Dateneingabe gegebenenfalls unterbrochen beziehungsweise pausiert und die Dateneingabe über die Schnittstelle höherer Priorität wird begonnen. Der Vorgang der Unterbrechung der Dateneingabe über die Schnittstelle niedrigerer Priorität kann dabei etwas Zeit, also mehrere Sekunden bis Minuten, in Anspruch nehmen. Soll über eine Schnittstelle höherer Priorität eine Dateneingabe begonnen werden oder ein Dateneingabemodus gestartet werden, so bleibt die Schnittstelle der höheren Priorität so lange gesperrt, bis die Dateneingabe beziehungsweise der Dateneingabemodus über die Schnittstelle niedrigerer Priorität unterbrochen oder gegebenenfalls beendet wurde. Während dieser Zeitperiode kann an die Gegenstelle der Schnittstelle höherer Priorität eine Meldung gegeben werden, die über den anhaltenden Unterbrechungsvorgang informiert. Ob die Dateneingabe über die Schnittstelle mit jeweils niedrigerer Priorität unterbrochen oder pausiert wird oder auch möglicherweise zunächst zu Ende geführt wird, bevor über die Schnittstelle der höheren Priorität Dateneingaben erfolgen können, kann beispielsweise auch von der jeweiligen Dateneingabe abhängen. Eine direkte Priorisierung der jeweiligen Dateneingabe ist dabei ebenfalls denkbar, kann aber auch beispielsweise einer einfachen Logik folgen, nach der Dateneingabe mit "darf abgebrochen werden" oder "darf nicht abgebrochen werden" markiert werden. So können zum Beispiel laufende Firmware-Aktualisierungen mit einer Markierung versehen werden, dass diese nicht abgebrochen werden dürfen, da ein Abbruch der Aktualisierung möglicherweise zu Datenverlust oder Schäden am Beatmungsgerät führen kann.

In manchen Ausführungsformen wird die Dateneingabe über die Schnittstelle niedrigerer Priorität unterbrochen, sobald eine Dateneingabe durch über eine Schnittstelle höherer Priorität aktiviert wird, auch wenn noch keine konkreten Dateneingaben erfolgen. In manchen Ausführungsformen bedeutet dies zum Beispiel, dass eine Berührung oder Aktivierung der Benutzerschnittstelle dazu führen kann, dass die Dateneingaben über andere Schnittstellen unterbrochen werden. In manchen Ausführungsformen wird die Dateneingabe über Schnittstellen niedrigerer Priorität aber auch erst unterbrochen, wenn eine konkrete Dateneingabe über eine Schnittstelle begonnen wird. Weiter ist es auch denkbar, dass vor der eigentlichen Dateneingabe ein Dateneingabemodus aktiviert werden muss. So kann eine Verbindung zwischen Beatmungsgerät und Gegenstelle über eine Schnittstelle höherer Priorität aufgebaut oder aktiviert werden ohne, dass eine Dateneingabe über die Schnittstelle niedrigerer Priorität unterbrochen wird. Die Unterbrechung folgt erst, wenn durch das aktivieren eines Dateneingabemodus über die Schnittstelle höherer Priorität eine konkrete Dateneingabe begonnen werden soll.

In manchen Ausführungsformen kann die Dateneingabe auch anhand des Typs der Dateneingabe gesperrt beziehungsweise freigegeben werden. Auch ist denkbar, dass lediglich bestimmte Typen von Dateneingaben unter die Handhabung der Priorität fallen. So kann beispielsweise durch die mit der Priorität zusammenhängenden Sperrung der Dateneingabe über andere Schnittstellen als der gerade aktiven Schnittstelle verhindert werden, dass Dateneingaben vorgenommen werden, welche unterschiedliche, eventuell auch gegensätzliche, Wirkungen erzielen.

Zudem können einige Typen von Dateneingaben, etwa die Datenübertragung von Sensoren, völlig von der Prioritätsbehandlung ausgenommen werden oder es wird ermöglicht, dass diese Dateneingaben nicht abgebrochen werden, wenn eine Dateneingabe über eine Schnittstelle höherer Priorität gestartet wird.

Auch bestimmte Aktionen beziehungsweise Dateneingaben über die Benutzerschnittstelle können von der Prioritätsbehandlung ausgeschlossen werden, sodass bestimmte Dateneingaben immer über die Benutzerschnittstelle ermöglicht werden. Dies bezieht sich insbesondere auf eine Menüführung oder auch die Bestätigung/Aktivierung bestimmter Vorgänge und/oder Dateneingaben über andere Schnittstellen.

In manchen beispielhaften Ausführungsformen sind die Schnittstellen mit einer Steuereinheit verbunden, welche differenziert, ob die über die Schnittstelle empfangene Eingabe eine zulässige Dateneingabe darstellt und darauf basierend eine Verbindung zwischen Schnittstelle und Beatmungsgerät oder einem der Bestandteile (z.B. Module, Modulgruppen) erlaubt oder gesperrt. Dazu hat die Steuereinheit Zugriff auf die Prioritätszuordnung der Schnittstellen oder die Prioritäten sind in der Steuereinheit hinterlegt. Die Steuereinheit kann dann anhand verschiedener Kriterien entscheiden, ob und/oder über welche Schnittstelle eine Dateneingabe oder der Start eines Dateneingabemodus erlaubt ist und durchgeführt werden kann.

In manchen beispielhaften Ausführungsformen bestimmt nicht nur die Priorität der Schnittstelle und ob ein Dateneingabe stattfindet darüber, ob ein Zugriff über eine Schnittstelle - also eine Dateneingabe, aber auch das Auslesen von Daten (die Datenausgabe) - möglich ist, sondern auch ob beispielsweise derzeit eine Beatmungstherapie durchgeführt wird und das Beatmungsgerät an einem Patienten verwendet wird. Ist das Beatmungsgerät aktiv an einem Patienten in Benutzung, also im laufenden Betrieb, so können beispielsweise einige Schnittstellen allgemein gesperrt werden. Dadurch kann beispielsweise eine Gefährdung während der Therapie durch Funkstrahlung, Überlastung des Prozessors oder durch einen Geräte-Neustart verhindert werden. Eine weitere Gefährdungsquelle kann durch das Aufspielen einer Firmware-Aktualisierung ausgemacht werden. Hierdurch kann es beispielsweise auch zu veränderter Arbeitsweise des Prozessors kommen, was wiederum negative Auswirkung auf die Funktion des Beatmungsgeräts haben kann. Daher kann es beispielsweise eingerichtet sein, dass einige Schnittstellen während der Anwendung des Beatmungsgeräts am Patienten vollständig für den Zugriff, also auch für das Auslesen von Daten, auf das Beatmungsgerät gesperrt werden können. Dabei ist unter anderem an kritische Schnittstellen wie für Mobilfunk-Verbindungen und/oder drahtlose Internet/WLAN-Verbindungen zu denken. Weniger kritische Schnittstellen, wie zum Beispiel WPAN-Verbindungen (Bluetooth), oder Netzwerkverbindungen für eine Fernsteuerung in zum Beispiel Schlaflaboren, können dabei von der allgemeinen Sperre ausgenommen sein. In einer alternativen Ausführungsform können beispielsweise auch einzelne Komponenten gegenüber Dateneingaben oder Schnittstellen gesperrt werden. So kann beispielsweise eine Dateneingabe durch die Fernüberwachung beziehungsweise Telemonitoring während der Therapie gesperrt werden, während ein Auslesen der Daten möglich bleibt.

Beispielsweise ist es in manchen Ausführungsformen möglich, dass der Beginn einer Dateneingabe über Schnittstellen höherer Priorität etwaige Dateneingabe über Schnittstellen niedrigerer Priorität unter- und/oder abbrechen kann. Darüber hinaus ist es in weiteren beispielhaften Ausführungsformen möglich, dass der gesamte Zugriff über die Schnittstelle niedrigerer Priorität das Beatmungsgerät unter- und/oder abgebrochen wird. Je nach aktueller Dateneingabe über die Schnittstelle der niedrigeren Priorität kann diese Unterbrechung beziehungsweise das Abbrechen einige Sekunden bis mehrere Minuten dauern, während denen die Unterbrechung initiiert wird. In dieser Wartezeit kann der Schnittstelle mit höherer Priorität zunächst noch der Zugriff verwehrt werden, bis die Unterbrechung der vorherigen Dateneingabe über die Schnittstelle niedrigerer Priorität durchgeführt ist. In manchen Fällen kann die Unterbrechung beispielsweise auch nicht eingeleitet werden, also der Dateneingabe über die Schnittstelle niedrigerer Priorität kann nicht unter- oder abgebrochen werden, obwohl der Schnittstelle mit der neueren Anfrage für einen Zugriff und/oder Dateneingabe eine höhere Priorität zugeordnet ist. Dies kann beispielsweise der Fall sein, wenn über eine Schnittstelle niedrigerer Priorität eine Firmware-Aktualisierung aufgespielt wird. In einem solchen Fall wäre die Fertigstellung der Dateneingabe der Schnittstelle niedrigerer Priorität abzuwarten, bevor mit der Dateneingabe über die Schnittstelle höherer Priorität begonnen werden kann. Dazu kann es beispielsweise eine zusätzliche Prioritätsverwaltung für einzelne Dateneingabe geben. Eine andere Möglichkeit kann zum Beispiel auch das Vergeben von Markierungen an die einzelne Dateneingabe sein, nach denen entschieden werden kann, ob eine Unterbrechung oder ein Abbrechen erlaubt ist, wenn eine neuere Dateneingabe über eine Schnittstelle höherer Priorität begonnen werden soll.

Eine beispielhafte Ausführungsform umfasst eine Bestätigung von Zugriffen und/oder Dateneingaben und/oder bestimmter Aktionen über eine Schnittstelle auf das Beatmungsgerät über eine andere Schnittstelle. Soll beispielsweise über eine Schnittstelle für Internet- bzw. Netzwerk-Verbindungen (Fernüberwachung/Telemonitoring/Fernwartung) eine Firmware-Aktualisierung auf das Beatmungsgerät bzw. ein Modul aufgespielt werden, kann es entsprechend nötig sein, dass diese Art der Dateneingabe beispielsweise über die Benutzerschnittstelle am Beatmungsgerät bestätigt und/oder akzeptiert wird. Auch bei der Bestätigung kann die Priorität der Schnittstellen berücksichtigt werden. So kann es zum Beispiel eingerichtet sein, das einzelne Dateneingaben über eine Schnittstelle durch eine Schnittstelle höherer Priorität bestätigt werden müssen. Dabei kann auch entschieden werden, ob alle Schnittstellen höherer Priorität dazu genutzt werden können, den Dateneingabe oder auch einen Zugriff allgemein, zu bestätigen. Beispielsweise wird über alle aktive Schnittstellen mit einer höheren Priorität als die Schnittstelle, über die ein Zugriff erfolgen soll, eine Bestätigung angefordert. Dabei reicht beispielsweise die Bestätigung über eine der Schnittstellen bereits aus, damit der Zugriff erlaubt wird. Alternativ ist es auch denkbar, dass eine Bestätigung bestimmter Zugriffe nur über die Schnittstelle oder Schnittstellen höchster Priorität möglich ist. Diese Aktionen, Dateneingaben und/oder Zugriffe können beispielsweise sensible Daten wie Patienteninformationen beinhalten. Auch eine Änderung der Therapiedaten und/oder Therapieeinstellungen kann eine Bestätigung über eine andere Schnittstelle, optional eine Schnittstelle höherer Priorität, erfordern. Alternativ oder ergänzend kann vorgesehen sein, dass zumindest teilweise eine Datenausgabe über eine Schnittstelle die Freigabe und/oder Bestätigung über eine Schnittstelle höherer Priorität erfordert. Soll beispielsweise über einen Fernzugriff, etwa über eine drahtlose Schnittstelle, auf sensible Daten zugegriffen werden, kann vorgesehen sein, dass über die Benutzerschnittstelle eine Bestätigung des Datenaustauschs bzw. der Datenausgabe angefordert wird.

Ferner kann die Freigabe von Zugriffen auch dazu verwendet werden, um eine zeitgleichen Dateneingabe über zwei Schnittstellen zu ermöglichen. Findet beispielsweise eine Dateneingabe über eine Schnittstelle hoher Priorität statt und über eine Schnittstelle niedrigerer Priorität soll ebenfalls eine Dateneingabe begonnen werden, so kann über die Schnittstelle höherer Priorität eine Freigabe für die Schnittstelle niedrigerer Priorität erteilt werden. Diese Freigabe kann beispielsweise auch nur einen sehr engen Rahmen umfassen, beispielsweise nur einzelne Bestandteile des Beatmungsgeräts, damit zum Beispiel die Dateneingabe über die Schnittstelle höherer Priorität nicht beeinflusst wird.

Auch die Anzeige einer Information bzw. Meldung zu Zugriffen zum Beispiel zwecks Dateneingabe/Datenausgabe über jeweilige Schnittstellen auf das Beatmungsgerät kann eine Information oder Meldung an andere Schnittstellen, insbesondere an Schnittstellen hoher Priorität und dabei besonders an die Benutzerschnittstelle am Beatmungsgerät, generiert und angezeigt werden. In einer weiteren Ausführungsform wird eine solche Information bzw. Meldung aber nur dann generiert, wenn das Beatmungsgerät nicht aktiv an einem Patienten angewendet wird, sich also im laufenden Betrieb befindet. Findet eine Dateneingabe/Datenausgabe während des laufenden Betriebs statt, kann eine Information bzw. Meldung darüber auch gespeichert werden und nach Beendigung des laufenden Betriebs angezeigt werden. Die Information bzw. Meldung kann dabei beispielsweise zu Beginn des Zugriffs angezeigt werden, aber auch während des gesamten Zugriffs angezeigt werden. Darüber hinaus ist auch eine Anzeige nach erfolgtem Zugriff beziehungsweise der Dateneingabe denkbar. Die Anzeige der Meldung kann beispielsweise für einen bestimmten Zeitraum nach dem Zugriff bestehen bleiben und dann automatisch ausgeblendet werden. Auch eine Aufforderung zur Bestätigung der Information über den Zugriff kann implementiert sein. Beispielsweise kann außerhalb der Anwendung der Therapie eine Dateneingabe über eine Schnittstelle erfolgen woraufhin eine Meldung an dem Beatmungsgerät beispielsweise für die Benutzerschnittstelle generiert und angezeigt wird. Der Anwender am Beatmungsgerät hat dort die Möglichkeit diese Meldung wahrzunehmen und die Meldung zu bestätigen. Bei einer Meldungsdauer, welche über die gesamte Dateneingabe anhalten soll, kann die Meldung, möglicherweise auch in einer abgewandelten Ausprägung, dauerhaft über die Benutzerschnittstelle angezeigt werden. Alternativ oder zusätzlich kann nach Abschluss der Dateneingabe eine (weitere) Meldung an die Benutzerschnittstelle weitergegeben werden. Diese Meldung kann beispielsweise auch eine Zusammenfassung oder Übersicht über die Veränderungen durch die Dateneingabe enthalten. Die Meldung kann Informationen über die verbundenen bzw. aktiven Schnittstellen, Schnittstellen über welche ein Datenaustausch (Dateneingabe und/oder Datenausgabe) erfolgt, den Inhalt des Datenaustauschs, die Dauer des Datenaustauschs, aktuell gesperrte und/oder nicht-gesperrte Schnittstellen und/oder auch ob sich das Beatmungsgerät im laufenden Betrieb befindet. Auch weitere Details über aktive Dateneingaben, Datenausgaben und Schnittstellen können in einer Meldung wiedergegeben werden.

Eine Datenausgabe sollte zu jeder Zeit, auch unabhängig der Sperrung von Dateneingaben, über zumindest eine oder zumindest zwei Schnittstellen möglich sein. Ist die Dateneingabe über eine Schnittstelle nicht möglich muss aber gleichzeitig die Datenausgabe über diese Schnittstelle nicht gesperrt werden. Über die Schnittstelle kann also eine Datenausgabe an die Gegenstelle erfolgen, es kann aber keine Dateneingabe von der entfernten Gegenstelle erfolgen. Unter der Möglichkeit Daten auszugeben ist dabei zu verstehen, dass dies möglich sein soll, aber nicht zwingend auch erfolgen muss. Es müssen also nicht zu jeder Zeit Daten über zumindest eine oder zumindest zwei Schnittstellen ausgelesen werden. So kann zu einer Zeit auch überhaupt kein Auslesen von Daten erfolgen.

In manchen Ausführungsformen kann die Datenausgabe aber auch unter bestimmten Umständen gesperrt werden. So kann eine zu große Anzahl an aktiven Verbindungen über die Schnittstellen zur Datenausgabe (auch für eine Dateneingabe denkbar) die Systemressourcen, wie z.B. Prozessorkapazität, Datenbuskapazität, Arbeitsspeicher, etc., überlasten. Um dies zu verhindern kann die Datenausgabe über zumindest eine oder manche Schnittstellen gesperrt werden. Für manche Anwendungsfälle des Beatmungsgeräts, beispielsweise in einem Schlaflabor, bei dem das Beatmungsgerät ausschließlich ferngesteuert wird, kann es sinnvoll sein, dass nur über die Fernsteuerung Datenaus- und Dateneingaben aktiviert sind. So kann beispielsweise die Benutzerschnittstelle in Form eines (berührungsempfindlichen) Bildschirms ausgeschaltet bleiben, damit der Patient schlafen kann.

Während der Datenausgabe, zum Beispiel, wenn die während des laufenden Betriebs aufgezeichneten Daten des Beatmungsgerätes an eine Gegenstelle gesendet werden, kann es ebenfalls sinnvoll sein, andere Dateneingaben und Datenausgaben während der Übertragung zu sperren, damit die Prozessorkapazität vollständig für z.B. eine Verschlüsselung der Daten zur Verfügung steht.

Auch kann vorgesehen sein, dass die Priorität der Schnittstelle anhand des Vorgangs und/oder der Aktion, beispielsweise der Art der Dateneingabe, und/oder nach Benutzer an der Schnittstelle eine Priorität zugeordnet wird. Beispielsweise kann die Priorität der Schnittstelle, über welche ein Betreuer/Arzt eine Dateneingabe tätigt eine höhere Priorität zugeordnet werden als der Schnittstelle, über welche der Nutzer/Patient des Beatmungsgerätes eine Dateneingabe vornimmt. Auch bei gleichen Schnittstellen, zum Beispiel einer drahtlosen Schnittstelle, über welche sowohl Patient als auch Betreuer/Arzt Dateneingaben vornehmen können, kann je nach Benutzer priorisiert werden. Es kann zum Beispiel vorgesehen sein, dass eine drahtlose Schnittstelle, über welche ein Betreuer/Arzt eine Dateneingabe vornimmt eine höhere Priorität erhält als wenn der Patient über die drahtlose Schnittstelle eine Dateneingabe und/oder Datenausgabe beabsichtigt. Auch kann die Schnittstelle, über welche sich ein Betreuer/Arzt oder auch ein Service-Techniker mit dem Beatmungsgerät verbindet eine höhere Priorität zugeordnet bekommen als zum Beispiel die Benutzerschnittstelle, über welche der Patient auf das Beatmungsgerät zugreift.

Ein Vorgang, welchem zum Beispiel eine besonders hohe Priorität zugeordnet werden kann, sodass die Schnittstelle, über welche der Vorgang erfolgt ebenfalls eine erhöhte Priorität zugeordnet wird, ist beispielsweise ein Servicevorgang wie das Aufspielen bzw. Installieren einer Firmware. Die Priorität eines Servicevorgangs kann beispielsweise auch über die Priorität der Benutzerschnittstelle am Beatmungsgeräte liegen. Die Priorität der Schnittstellen kann also in der Form variabel sein, dass ein Vorgang, beispielsweise eine Dateneingabe bzw. die Art der Dateneingabe, die Priorität der Schnittstelle maßgeblich beeinflusst. Ein Vorgang besonders hoher Priorität kann beispielsweise das Umschalten vom nicht-laufenden Betrieb in den laufenden Betrieb sein. Startet der Patient beispielsweise seine Therapie, möchte das Beatmungsgerät also in den laufenden Betrieb schalten, kann diese Aktion/dieser Vorgang eine höhere Priorität zur Folge haben als beispielsweise ein Datenaustausch eines Betreuers/Arztes über eine drahtlose Schnittstelle, selbst wenn diese aufgrund der Nutzung durch den Betreuer/Arzt eigentlich eine höhere Priorität erhalten sollte als die Schnittstelle über welche der Patient/Benutzer agiert.

Auch können gewisse Vorgänge dazu führen, dass Schnittstellen zumindest teilweise gesperrt werden. So können neben einem Übergang zwischen laufenden und nicht-laufenden Betrieb auch weitere Vorgänge dazu führen, dass zumindest ein Teil der Schnittstellen zeitweise gesperrt werden. Diese Vorgänge können Bootup (z.B. Starten/Hochfahren des Gerätes) Initialisierung von Teilen der Elektronik, z. B. auch Schnittstellenmodulen, Firmware-Update, Verarbeitung von Daten, Selbsttest, Virusscan und Einleitung eines Shutdown (Ausschalten des Gerätes bzw. Herunterfahren) sein. Insbesondere die Änderung eines Betriebszustandes, wie zum Beispiel das Umschalten zwischen laufendem und nicht-laufendem Betrieb und/oder das Einschalten des Beatmungsgerätes und/oder das Ausschalten des Beatmungsgerätes, kann eine zumindest zeitweise bzw. vorrübergehende Sperre einiger oder aller Schnittstellen bewirken.

Das erfinderische Beatmungsgerät besitzt beispielsweise zumindest drei Schnittstellen, in manchen Ausführungsformen auch zumindest vier oder fünf Schnittstellen. Besitzt das Beatmungsgerät zumindest fünf Schnittstellen, sollte jede der folgenden Gruppen a, b, c, d, e durch zumindest eine Schnittstelle repräsentiert werden:
a. drahtlose Schnittstelle für große Entfernungen (Mobilfunk) zum Beispiel für Telemonitoring, Teleservice, Telesetting, FW-Update
b. drahtlose Schnittstelle für kleine Entfernungen (Bluetooth, WPAN) zum Beispiel für Fernbedienung über App, Feedback zur Therapie in App, Service-Prozesse; Verbindung zu Diagnose-Geräten (Polygraphie), FW-Update/Firmware-Aktualisierung)
c. Benutzerschnittstelle (User-Interface, GUI) zum Beispiel für Bedienung, Feedback, Service
d. Kabel-gebundene Schnittstellen zum Beispiel für Fernsteuerung im Schlaflabor, Auslesen detaillierter Therapiedaten, Verbindung zu Diagnose-Geräten (PSG), externer Anschluss eines Funkmoduls, Service, Kommunikation mit dem Prüfstand, FW-Update
e. Speicher-Medium zum Beispiel für Auslesen detaillierter Therapiedaten, Programmierung durch Provider bei der Geräte-Ausgabe, FW-Update/Firmware-Aktualisierung)

Bei weniger als fünf Schnittstellen sind die Gruppen a, c, d, e zu bevorzugen, während bei einem Beatmungsgerät mit nur drei Schnittstellen zumindest die Gruppen c, d, e enthalten sein sollen. Für manche Ausführungsformen kann hier auch eine andere Bevorzugung von Schnittstellenarten getroffen werden.

Weist das Beatmungsgerät beispielsweise zwei Schnittstellen zur drahtlosen Verbindung über weite Entfernung auf, beispielsweise ein internes und ein externes Modem, so kann das es eingerichtet sein, dass beispielsweise das interne Modem komplett deaktiviert wird, bis das externe Modem entfernt und nicht mehr mit dem Beatmungsgerät verbunden bzw. ein Teil des Beatmungsgerätes ist.

Weist das Beatmungsgerät zum Beispiel drahtlose Schnittstellen auf, sollten diese beispielsweise einzeln aktiviert und deaktiviert werden können. In manchen Ausführungsformen sollten alle anderen Schnittstellen gesperrt sein, wenn eine Eingabe über die Benutzerschnittstelle am Gerät getätigt wird. Die Benutzerschnittstelle hat demnach also immer die höchste Priorität aller Schnittstellen. Die restlichen Schnittstellen besitzen in manchen Ausführungsformen unter einander ebenfalls eine Priorität. Diese Priorität soll weiter dazu dienen, dass immer nur eine Schnittstelle für Eingaben, wie beispielsweise Änderungen oder Dateneingaben am Gerät, freigeschaltet ist. Die anderen Schnittstellen sind währenddessen also gesperrt. Unabhängig von der Freischaltung bezüglich von Eingaben, ist in einigen Ausführungsformen des Geräts ein Auslesen von Daten jederzeit über zumindest zwei Schnittstellen möglich.

An dem Beatmungsgerät ist es nicht zwangsläufig so eingestellt, dass alle Dateneingaben über die zu einer Zeit nicht aktiven Schnittstellen gesperrt werden. Beispielsweise könnten Daten aus einem externen Sensor oder einem Diagnosegerät, etc., weiterhin in das Beatmungsgerät eingegeben und geschrieben werden. Diese Dateneingabe soll auch weiterhin stattfinden können, während beispielsweise Dateneingaben zur Einstellung einer Therapie über die Benutzerschnittstelle erfolgt. So sollte hier lediglich verhindert werden, dass eine Dateneingabe für Datentypen gesperrt wird, welche mit der Dateneingabe über die Schnittstelle mit der derzeitig höchsten Priorität im Konflikt stehen.

Die Reihenfolge der Priorität kann beispielsweise im Quellcode der entsprechenden Firmware fest hinterlegt werden. In bestimmten Fällen kann dem Anwender auf Seiten der Gegenstelle an einer Schnittstelle höherer Priorität auch darüber entscheiden, ob ein Abbrechen oder Unterbrechen des laufenden Vorgangs, also der aktuellen Dateneingabe über eine Schnittstelle niedrigerer Priorität, erwünscht ist und durchgeführt werden soll.

Das Beatmungsgerät kann bei einer Dateneingabe auch nur so gegenüber Dateneingaben anderer Schnittstellen gesperrt werden, dass lediglich die Bestandteile, zum Beispiel zusammengefasst in Module, gesperrt werden, welche von der Dateneingabe betroffen sind. So können beispielsweise dennoch Dateneingaben über verschiedene Schnittstellen gleichzeitig erfolgen, lediglich nicht für einen Bestandteil bzw. ein Modul über zwei oder mehr Schnittstellen gleichzeitig.

Teilweise kann ein Atmen in die Maske z. B. den Therapiestart auslösen, wodurch z. B. eine Modem-Kommunikation (drahtlose Schnittstelle für große Entfernungen oder auch kabelgebundene Schnittstelle) abgebrochen wird, um eine Interaktion der Funkstrahlung mit der Elektronik des Gerätes oder mit dem Patienten zu vermeiden - oder auch um eine Überlastung des Controllers mit zu vielen parallelen Aufgaben zu vermeiden.

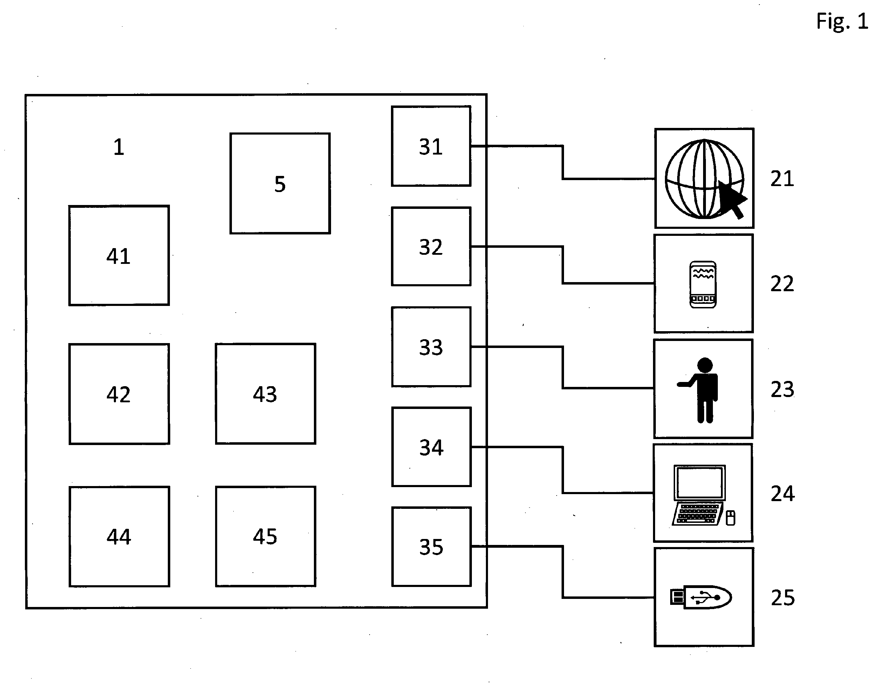

Im Folgenden wird die Erfindung anhand der Figuren 1 bis 3 über beispielhafte Ausführungsformen näher erläutert.

Die Figur 1 zeigt dabei den beispielhaften, schematischen Aufbau eines Beatmungsgeräts 1 mit den Schnittstellen 31, 32, 33, 34, 35 sowie den Modulen 41, 42, 43, 44, 45.

Beispielsweise ist Modul 41 als Gebläse- und/oder Ventileinheit ausgebildet um einen Atemgasluftstrom zur Beatmung bzw. Therapie eines Patienten zu erzeugen.

Modul 42 ist beispielsweise als Sensoreinheit eingerichtet, welche Messwerte, insbesondere Parameter, die mit einem Atemfluss, einem Atemvolumen, einer Atemfrequenz, einer Einatmungs- und Ausatmungsdauer, einer Atemkontur, einer Leckage oder einem Therapiedruck in Zusammenhang stehen, zu erfassen. Optional kann die Sensoreinheit 42 zusätzliche Messungen von Bestandteilen oder Temperatur des Atemgases oder des Blutes vornehmen. Das als Sensoreinheit ausgebildete Modul 42 übermittelt die erfassten Messwerte an das als beispielsweise Aufbereitungseinheit ausgebildete Modul 43.

Das als Aufbereitungseinheit ausgebildete Modul 43 kann die erfassten Messwerte aufbereiten. Beispielsweise kann die Aufbereitungseinheit eine Glättung, eine Artefaktbereinigung oder ein Downsampling der Messwerte durchführen. In manchen Ausführungsformen ist das Modul 43 auch als kombinierte Aufbereitungs-, Berechnungs- und Erkennungseinheit ausgebildet. Die Berechnungseinheit berechnet aus den durch die Sensoreinheit erfassten und durch die Aufbereitungseinheit aufbereiteten Messwerten Signale und/oder Kenngrößen, wie beispielsweise einen Mittelwert, einen Median, ein Perzentil, eine Ableitung, eine Häufigkeitsverteilung, eine Dauer oder einen Anteil eines Über- oder Unterschreitens von Schwellenwerten. Die Erkennungseinheit ist eingerichtet, Ereignisse/Zustände wie beispielsweise Alarme, Atemaussetzer, Artefakte, Hustenstöße, Sauerstoff(ent)sättigungen, asynchrone zwischen Gerät und Anwender, Einatmen, Ausatmen oder mandatorische Atemzüge zu erkennen.

Das Modul 44 ist beispielsweise als interne Speichereinheit und/oder als Zwischenspeichereinheit ausgebildet, welche unter anderem die durch das Modul 42 erfassten Werte/Parameter und/oder die durch das Modul 43 aufbereiteten Werte, Daten und/oder Informationen speichert beziehungsweise zumindest zwischenspeichert. Eine Zwischenspeicherung bedeutet zum Beispiel, dass die Werte, Daten und/oder Informationen bis zu einer Übertragung gespeichert und danach zum Beispiel gelöscht oder zum Überschreiben freigegeben werden.

Das Modul 45 kann beispielsweise als eine Überwachungseinheit ausgebildet sein, welche technische Probleme des Beatmungsgerätes 1 erfasst. Technische Probleme können beispielsweise ein niedriger Akkustand, Fehler in der Elektronik, ein defekter Akku, ein defektes Bauteil, ein Stromausfall, ein nicht korrekt funktionierendes Zubehörteil, ein unplausibler Messwert oder ein Verlassen eines erlaubten Temperaturbereichs sein. Die Überwachungseinheit kann bei einem erkannten technischen Problem einen Alarm auf dem Beatmungsgerät 1 über eine der Schnittstellen anzeigen oder übermitteln. In manchen Ausführungsformen können diese technischen Probleme zum Teil auch als schwerwiegende Fehler angesehen werden, welche außer einer entsprechenden Fehlermeldung am Display (Benutzerschnittstelle) andere Dateneingaben und Datenausgaben sperrt.

In weiteren beispielhaften Ausführungsformen können die Module weiter in Modulgruppen zusammengefasst werden, wobei sich diese Modulgruppen durchaus überschneiden können. Zur Verdeutlichung, wie mit Modulgruppen umgegangen werden kann, werden an dieser Stelle die Module 41 und 42, die Module 42 und 43, sowie die Module 42 und 44 und 45 zu jeweils einer Modulgruppe zusammengefasst, welche nicht näher in den Figuren verdeutlicht sind

Ferner verfügt das Beatmungsgerät über eine Steuereinheit 5, welche mit den Schnittstellen 31, 32, 33, 34, 35 und den Modulen 41, 42, 43, 44, 45 verbunden ist. Die Steuereinheit 5 ist dazu eingerichtet, unter anderem die einzelnen Module 41, 42, 43, 44, 45 sowie die Schnittstellen 31, 32, 33, 34, 35 zu steuern und optional auch Daten innerhalb des Beatmungsgeräts zu übertragen - zwischen einzelnen Modulen oder auch zwischen Modulen und Schnittstellen - oder zumindest die Kommunikation zwischen Schnittstelle und Modul zu ermöglichen und zu steuern. Die Steuereinheit 5 verfügt dazu auch oder hat Zugriff auf die Prioritätenzuordnung der einzelnen Schnittstellen. Auch ist die Steuereinheit eingerichtet, das Beatmungsgerät 1 in den laufenden Betrieb beziehungsweise in den nicht-laufenden Betrieb übergehen zu lassen und/oder diesen Übergang zu steuern.

Die Schnittstellen sind dabei so eingerichtet und ausgebildet, dass sie Verbindungen zu den entfernten Gegenstellen 21 (Internet), 22 (Smartphone), 23 (Anwender), 24 (Computer), 25 (USB Stick) aufbauen können. Die Schnittstelle 31 wäre beispielsweise eine drahtlose Schnittstelle für große Entfernungen (hier z.B. WiFi/WLAN), die Schnittstelle 32 eine drahtlose Schnittstelle für kurze Entfernungen (hier z.B. Bluetooth), die Schnittstelle 33 eine Benutzerschnittstelle, die Schnittstelle 34 eine kabelgebundene Schnittstelle und die Schnittstelle 35 eine Schnittstelle für Speichermedien (hier z.B. eine USB Schnittstelle).

Die Schnittstelle 31 ist beispielsweise als drahtlose Schnittstelle für große Entfernungen ausgebildet, um beispielsweise eine Verbindung mit dem Internet 21 aufzubauen. Die Verbindung zum Internet kann dabei zum Beispiel über den Mobilfunk, eine drahtlose Netzwerkverbindung wie WLAN/Wifi oder zum Beispiel über ein Kabelmodem erfolgen.

Die Schnittstelle 32 ist eingerichtet eine Verbindung über beispielsweise ein WPAN, wie zum Beispiel Bluetooth, mit einem Smartphone 22 aufzubauen. Auch andere Geräte, wie zum Beispiel ein Tablet-PC oder aber auch Sensoren mit drahtlosen Verbindungen sind zur Verbindung mit drahtlosen Schnittstellen für kurze Entfernungen denkbar.

Die Schnittstelle 33 ist beispielsweise als Benutzerschnittstelle am Beatmungsgerät 1 eingerichtet und ermöglicht die Kommunikation beziehungsweise Verbindung mit einem Anwender 23. Die Schnittstelle 33 kann beispielsweise als berührungsempfindlicher Bildschirm (Touch-Screen) ausgeführt sein, welcher gleichzeitig die Anzeige bzw.

Datenausgabe von Daten/Informationen etc. aber auch die Dateneingabe ermöglicht. Als Benutzerschnittstelle wären aber auch Druck-, Dreh- und/oder Kippschalter möglich.

Die Schnittstelle 34 ist beispielsweise eingerichtet, um eine Verbindung zu einem Computer 24, beispielsweise als Diagnosewerkzeug, aufzubauen. Die Schnittstelle 34 ist beispielsweise eingerichtet, um eine Verbindung zu einem Gebäudenetzwerk 24 - beispielsweise in einem Schlaflabor oder einem Krankenhaus - aufzubauen. Die Schnittstelle 35 ist beispielsweise eingerichtet um eine Verbindung mit dem Internet 21 aufzubauen. Die Verbindung zum Internet kann dabei zum Beispiel über den Mobilfunk, eine drahtlose Netzwerkverbindung wie WLAN/Wifi oder zum Beispiel über ein Kabelmodem erfolgen. Das Beatmungsgerät ist insgesamt so eingerichtet und ausgebildet, dass eine Kommunikation aller Schnittstellen mit allen Modulen möglich ist. Auch eine Kommunikation der Schnittstellen untereinander und der Module untereinander wird als gegeben vorausgesetzt. Es ist darauf hinzuweisen, dass das Beatmungsgerät 1 im Sinne der Erfindung auch eine größere oder auch niedrigere Anzahl an Schnittstellen aufweisen kann. Auch die Art der Schnittstellen kann von den hier genannten abweichen oder auch zusätzlich erweitert werden.

Beispielsweise werden den Schnittstellen Prioritäten zugeordnet, welche aufgrund der maximalen oder prinzipiellen Entfernung basieren. Demnach wird der Schnittstelle 33 die höchste Priorität zugeordnet, unter anderem, da die Schnittstelle 33 als Benutzerschnittstelle direkt am Beatmungsgerät ausgebildet ist. Die nächste Priorität kann der Schnittstelle 32 zugeordnet werden, welche als drahtlose Schnittstelle für kurze Entfernungen eingerichtet ist. Folgend wird der Schnittstelle 35 die dritthöchste Priorität zugeordnet. Der Schnittstelle 34 kann eine niedrigere Priorität zugeordnet werden wie der Schnittstelle 33, aber eine höhere Priorität als der Schnittstelle 31. Die Schnittstelle 31 würde in dem beschriebenen Ausführungsbeispiel also die niedrigste Priorität erhalten. Insgesamt ergibt sich also die Prioritätsreihenfolge von hoher zu niedriger Priorität: 33 - 32 - 35 - 34 - 31. Die Reihenfolge der Priorität kann beispielsweise auch manuell angepasst werden oder anhand anderer Kriterien erfolgen. In manchen Ausführungsformen wird die Priorität der Schnittstelle 33, welche als Benutzerschnittstelle am Beatmungsgerät 1 ausgeführt ist, als die höchste Priorität festgesetzt.

Folgend sollen einige Fallbeispiele das Verfahren zur Auswirkung der vergebenen Priorität verdeutlichen.

Findet beispielsweise eine Dateneingabe an Modul 41 durch den Anwender 23 an der Schnittstelle 33 statt, so kann zumindest an dem Modul 41 über die Schnittstellen 32, 31, 34 und 35 keine Dateneingabe stattfinden oder begonnen werden. Zum einen resultiert dies daraus, dass eine Dateneingabe an einem Modul immer nur über eine Schnittstelle gleichzeitig möglich sein soll, zum anderen spielt hier auch die Priorität der Schnittstelle 33 eine Rolle. So ist beispielsweise eine Dateneingabe über Schnittstellen niedrigerer Priorität nur dann möglich, soweit keine Dateneingabe über eine Schnittstelle einer höheren Priorität erfolgt. Beispielsweise kann durch die Dateneingabe auch das gesamte Beatmungsgerät 1 gegenüber Dateneingaben über andere Schnittstellen gesperrt werden oder weitere Dateneingaben über andere Schnittstellen gesperrt werden. Neben der Sperrung von Dateneingaben über andere Schnittstelle, kann gleichzeitig für einige Schnittstellen der Zugriff allgemein - also auch ein Auslesen von Daten - gesperrt werden. Neben der Sperrung einzelner Module oder des gesamten Beatmungsgeräts gegenüber weiteren Dateneingaben über andere Schnittstellen, können prinzipiell auch Modulgruppen gesperrt werden. In dem hier beschriebenen Fallbeispiel einer Dateneingabe an Modul 41 über Schnittstelle 33 können zum Beispiel die Modulgruppen, welchen Modul 41 angehört, gegenüber Dateneingaben über andere Schnittstellen gesperrt werden - in der weiter vorne aufgeführten Gruppierung wäre also die Modulgruppe beinhaltend Modul 41 und 42 gegenüber Dateneingaben gesperrt. In manchen Ausführungsformen können unter bestimmten Kriterien, zum Beispiel während einer aktiven Therapie mit dem Beatmungsgerät an einem Patienten, auch Dateneingabe und/oder Zugriffe auf das Beatmungsgerät über einige Schnittstellen generell gesperrt werden. Als Beispiel wäre hier ein Zugriff über die Schnittstelle 31, insbesondere wenn die Schnittstelle 31 als drahtlose Funkverbindung ausgelegt ist. In manchen Ausführungsformen kann hierfür eine Priorität anhand der möglichen Prozessorlast oder auch möglicher Belastung des Patienten (beispielsweise durch Funkstrahlung) vergeben werden.

Weiter kann dem Anwender 23 an der Schnittstelle 33 eine Information oder Meldung generiert und angezeigt werden, wenn zum Beispiel eine Dateneingabe über eine Schnittstelle mit niedrigerer Priorität begonnen werden soll. Sind die vom Dateneingabe betroffenen Module oder Modulgruppen nicht gesperrt, dann kann diese Meldung zu rein informativen Zwecken ausgegeben werden. Ist beispielsweise das Beatmungsgerät 1 generell gegenüber Dateneingaben über andere Schnittstellen gesperrt, so kann dem Anwender 23 an der Schnittstelle 33 auch eine Freigabemöglichkeit angezeigt werden, sodass beispielsweise einzelne Module für die Dateneingabe über eine Schnittstelle niedrigerer Priorität freigegeben werden. Zusätzlich ist es auch denkbar, dass über alle anderen Schnittstellen Meldungen erzeugt und ausgegeben werden können, wenn aktuell Dateneingabe vorgenommen werden. Alternativ kann beispielsweise aber auch nur über Schnittstellen höherer Priorität eine solche Meldung ausgegeben werden.

Wird in einem anderen Fall zum Beispiel eine Dateneingabe an Modul 42 über Schnittstelle 34 durch den Computer 24 vorgenommen, so wird dadurch zum Beispiel eine Dateneingabe zumindest über die Schnittstelle 31 gesperrt, da dieser eine niedrigere Priorität zugeordnet ist als der Schnittstelle 34. Das Sperren einer Dateneingabe kann, wie bereits im vorherigen Fall beschrieben, auf Ebene des Moduls, der Modulgruppe oder auch für das gesamte Beatmungsgerät 1 gelten. Auf Ebene der Modulgruppen wären bei einer Dateneingabe bei Modul 42 und den vorher beispielhaft definierten Modulgruppen alle Modulgruppen gesperrt, da Modul 42 als Teil aller Modulgruppen zugeordnet wurde. Während ein Dateneingabe über die Schnittstelle 31 gesperrt wird, kann eine Dateneingabe über die Schnittstellen 32, 33 und 35 prinzipiell möglich sein, da diesen eine höhere Priorität zugeordnet ist. Wird beispielsweise durch das Smartphone 22 über Schnittstelle 32 versucht eine Dateneingabe zu beginnen, so muss zunächst die Dateneingabe, welcher über Schnittstelle 34 erfolgt, abgebrochen oder unterbrochen werden. Je nach Dateneingabe kann eine gewisse Zeit in Anspruch genommen werden, bis der Prozess erfolgreich unter- oder abgebrochen werden kann. Für manche Dateneingabe kann beispielsweise zusätzlich auch gelten, dass diese grundsätzlich nicht unter- oder abgebrochen werden können. Dies ist zum Beispiel der Fall für Firmware-Aktualisierungen, bei denen ein Abbruch unter anderem auch zu Fehlfunktionen des Beatmungsgeräts führen können.

Während, zu Beginn und/oder auch nach Beendigung der Dateneingabe über Schnittstelle 34 kann eine Meldung oder Information beispielsweise über die Schnittstelle 33 ausgeben werden, welche über die Dateneingabe informiert. Insbesondere bei einer Meldung über die Dateneingabe nach Beendigung der Dateneingabe kann zusätzlich in der Meldung eine Zusammenfassung der durchgeführten Änderung ausgegeben werden.

Für manche Dateneingabe kann zusätzlich eine Bestätigung über die Schnittstelle höchster Priorität oder auch über eine andere Schnittstelle erforderlich sein.

In manchen Ausführungsformen steuert die Steuereinheit 5 die Sperrung der Schnittstellen und/oder die Dateneingaben über die jeweilige Schnittstelle. Wird eine Dateneingabe über eine Schnittstelle angestrebt oder soll begonnen werden - beispielsweise wird ein Dateneingabemodus gestartet - entscheidet die Steuereinheit anhand der Priorität, ob die Dateneingabe durchgeführt werden kann. Zudem steuert die Steuereinheit 5 auch den Übergang und die Umstellung des laufenden Betriebs in den nicht-laufenden Betrieb und umgekehrt. Soll mit einer Dateneingabe über eine Schnittstelle begonnen werden, so entscheidet die Steuereinheit 5 zum einen ob die Dateneingabe während des laufenden Betriebs möglich ist, ob die Dateneingabe nicht möglich ist oder auch ob das Beatmungsgerät in den nicht-laufenden Betrieb umgestellt wird, damit die Dateneingabe ermöglicht wird. In letzterem Fall sperrt die Steuereinheit die Dateneingabe so lange, bis die Umstellung von dem laufenden Betrieb in den nicht-laufenden Betrieb abgeschlossen ist. Diese Umstellung kann zum Beispiel auch das Speichern der während des Betriebs aufgenommenen Daten umfassen.

Über manche Module oder Bestandteile des Beatmungsgeräts, wie zum Beispiel über eine Überwachungseinheit oder auch die Steuereinheit kann geprüft werden, ob ausreichend Systemressourcen und eine sichere Stromversorgung vorliegt, damit Dateneingaben und Datenausgaben überhaupt ermöglicht werden. Sollten vorrübergehend nicht ausreichend Systemressourcen vorhanden sein, zum Beispiel, weil eine verschlüsselte Datenübertragung stattfindet, was ein hohes Maß an Prozessorkapazitäten bindet, kann so beispielsweise durch die Steuereinheit 5, optional in Zusammenhang mit Daten der Überwachungseinheit, alle anderen Schnittstellen für Dateneingaben und Datenausgaben gesperrt werden.

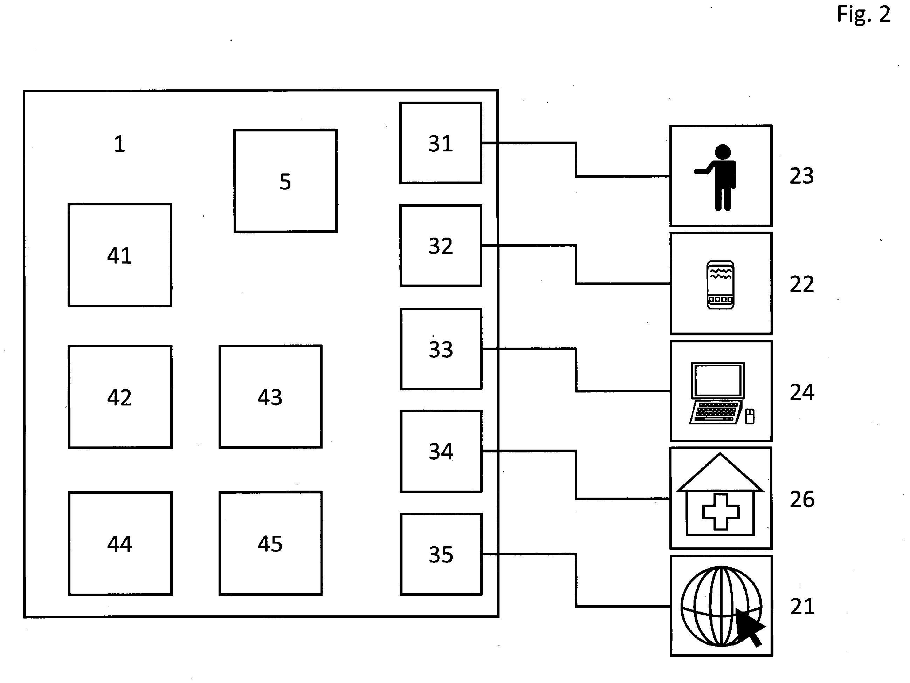

Die Figur 2 zeigt den beispielhaften, schematischen Aufbau eines Beatmungsgeräts 1 mit den Schnittstellen 31, 32, 33, 34, 35 sowie den Modulen 41, 42, 43, 44, 45.

Ferner verfügt das Beatmungsgerät über eine Steuereinheit 5, welche mit den Schnittstellen und den Modulen verbunden ist und kommuniziert. Die Steuereinheit 5 verfügt dazu auch oder hat Zugriff auf die Prioritätenzuordnung der einzelnen Schnittstellen. Die Schnittstellen sind dabei so eingerichtet und ausgebildet, dass sie Verbindungen zu den entfernten Gegenstellen 23 (Anwender), 22 (Smartphone), 24 (Computer), 26 (Gebäudenetzwerk), 21 (Internet) aufbauen können. In weiteren beispielhaften Ausführungsformen können die Module weiter in Modulgruppen zusammengefasst werden, wobei sich diese Modulgruppen durchaus überschneiden können. Zur Verdeutlichung, wie mit Modulgruppen umgegangen werden kann, werden an dieser Stelle die Module 41 und 42, die Module 42 und 43, sowie die Module 42 und 44 und 45 zu jeweils einer Modulgruppe zusammengefasst, welche nicht näher in den Figuren verdeutlicht sind. Die Schnittstelle 31 ist beispielsweise als Benutzerschnittstelle am Beatmungsgerät 1 eingerichtet und ermöglicht die Kommunikation beziehungsweise Verbindung mit einem Anwender 23. Die Schnittstelle 32 ist eingerichtet eine Verbindung über beispielsweise ein WPAN, wie zum Beispiel Bluetooth, mit einem Smartphone 22 aufzubauen. Die Schnittstelle 33 ist beispielsweise eingerichtet, um eine Verbindung zu einem Computer 24, beispielsweise als Diagnosewerkzeug, aufzubauen. Die Schnittstelle 34 ist beispielsweise eingerichtet, um eine Verbindung zu einem Gebäudenetzwerk 26 - beispielsweise in einem Schlaflabor oder einem Krankenhaus - aufzubauen. Die Schnittstelle 35 ist beispielsweise eingerichtet um eine Verbindung mit dem Internet 21 aufzubauen. Die Verbindung zum Internet kann dabei zum Beispiel über den Mobilfunk, eine drahtlose Netzwerkverbindung wie WLAN/Wifi oder zum Beispiel über ein Kabelmodem erfolgen. Das Beatmungsgerät ist insgesamt so eingerichtet und ausgebildet, dass eine Kommunikation aller Schnittstellen mit allen Modulen möglich ist. Auch eine Kommunikation der Schnittstellen untereinander und der Module untereinander wird als gegeben vorausgesetzt. Es ist darauf hinzuweisen, dass das Beatmungsgerät 1 im Sinne der Erfindung auch eine größere oder auch niedrigere Anzahl an Schnittstellen aufweisen kann. Auch die Art der Schnittstellen kann von den hier genannten abweichen oder auch zusätzlich erweitert werden.

Beispielsweise werden den Schnittstellen Prioritäten zugeordnet, welche aufgrund der maximalen oder prinzipiellen Entfernung basieren. Demnach wird der Schnittstelle 31 die höchste Priorität zugeordnet, unter anderem, da die Schnittstelle 31 als Benutzerschnittstelle direkt am Beatmungsgerät ausgebildet ist. Die nächste Priorität kann der Schnittstelle 32 zugeordnet werden, welche beispielsweise über. Folgend wird der Schnittstelle 33 die dritthöchste Priorität zugeordnet. Der Schnittstelle 34 kann eine niedrigere Priorität zugeordnet werden wie der Schnittstelle 33, aber eine höhere Priorität als der Schnittstelle 35. Die Schnittstelle 35 würde in dem beschriebenen Ausführungsbeispiel also die niedrigste Priorität erhalten. Insgesamt ergibt sich also die Prioritätsreihenfolge von hoher zu niedriger Priorität: 31 - 32 - 33 - 34 - 35. Die Reihenfolge der Priorität kann beispielsweise auch manuell angepasst werden oder anhand anderer Kriterien erfolgen. In manchen Ausführungsformen wird die Priorität der Schnittstelle 31, welche als Benutzerschnittstelle am Beatmungsgerät 1 ausgeführt ist, als die höchste Priorität festgesetzt.

Folgend sollen einige Fallbeispiele das Verfahren zur Auswirkung der vergebenen Priorität verdeutlichen.

Findet beispielsweise eine Dateneingabe an Modul 41 durch den Anwender 23 an der Schnittstelle 31 statt, so kann zumindest an dem Modul 41 über die Schnittstellen 32, 33, 34 und 35 keine Dateneingabe stattfinden oder begonnen werden. Zum einen resultiert dies daraus, dass eine Dateneingabe an einem Modul immer nur über eine Schnittstelle gleichzeitig möglich sein soll, zum anderen spielt hier auch die Priorität der Schnittstelle 31 eine Rolle. So ist beispielsweise eine Dateneingabe über Schnittstellen niedrigerer Priorität nur dann möglich, soweit keine Dateneingabe über eine Schnittstelle einer höheren Priorität erfolgt. Beispielsweise kann durch die Dateneingabe auch das gesamte Beatmungsgerät 1 gegenüber Dateneingaben über andere Schnittstellen gesperrt werden oder weitere Dateneingaben über andere Schnittstellen gesperrt werden. Neben der Sperrung von Dateneingaben über andere Schnittstelle, kann gleichzeitig für einige Schnittstellen der Zugriff allgemein - also auch ein Auslesen von Daten - gesperrt werden. Neben der Sperrung einzelner Module oder des gesamten Beatmungsgeräts gegenüber weiteren Dateneingaben über andere Schnittstellen, können prinzipiell auch Modulgruppen gesperrt werden. In dem hier beschriebenen Fallbeispiel einer Dateneingabe an Modul 41 über Schnittstelle 31 können zum Beispiel die Modulgruppen, welchen Modul 41 angehört, gegenüber Dateneingaben über andere Schnittstellen gesperrt werden - in der weiter vorne aufgeführten Gruppierung wäre also die Modulgruppe beinhaltend Modul 41 und 42 gegenüber Dateneingaben gesperrt. In manchen Ausführungsformen können unter bestimmten Kriterien, zum Beispiel während einer aktiven Therapie mit dem Beatmungsgerät an einem Patienten, auch Dateneingabe und/oder Zugriffe auf das Beatmungsgerät über einige Schnittstellen generell gesperrt werden. Als Beispiel wäre hier ein Zugriff über die Schnittstelle 35, insbesondere wenn die Schnittstelle 35 als drahtlose Funkverbindung ausgelegt ist. In manchen Ausführungsformen kann hierfür eine Priorität anhand der möglichen Prozessorlast oder auch möglicher Belastung des Patienten (beispielsweise durch Funkstrahlung) vergeben werden.

Weiter kann dem Anwender 23 an der Schnittstelle 31 eine Information oder Meldung generiert und angezeigt werden, wenn zum Beispiel eine Dateneingabe über eine Schnittstelle mit niedrigerer Priorität begonnen werden soll. Sind die vom Dateneingabe betroffenen Module oder Modulgruppen nicht gesperrt, dann kann diese Meldung zu rein informativen Zwecken ausgegeben werden. Ist beispielsweise das Beatmungsgerät 1 generell gegenüber Dateneingaben über andere Schnittstellen gesperrt, so kann dem Anwender 23 an der Schnittstelle 31 auch eine Freigabemöglichkeit angezeigt werden, sodass beispielsweise einzelne Module für die Dateneingabe über eine Schnittstelle niedrigerer Priorität freigegeben werden. Zusätzlich ist es auch denkbar, dass über alle anderen Schnittstellen Meldungen erzeugt und ausgegeben werden können, wenn aktuell Dateneingabe vorgenommen werden. Alternativ kann beispielsweise aber auch nur über Schnittstellen höherer Priorität eine solche Meldung ausgegeben werden.

Wird in einem anderen Fall zum Beispiel eine Dateneingabe an Modul 42 über Schnittstelle 33 durch den Computer 24 vorgenommen, so wird dadurch zum Beispiel eine Dateneingabe zumindest über die Schnittstellen 34 und 35 gesperrt, da diesen eine niedrigere Priorität zugeordnet ist als der Schnittstelle 33. Das Sperren einer Dateneingabe kann, wie bereits im vorherigen Fall beschrieben, auf Ebene des Moduls, der Modulgruppe oder auch für das gesamte Beatmungsgerät 1 gelten. Auf Ebene der Modulgruppen wären bei einer Dateneingabe bei Modul 42 und den vorher beispielhaft definierten Modulgruppen alle Modulgruppen gesperrt, da Modul 42 als Teil aller Modulgruppen zugeordnet wurde. Während ein Dateneingabe über die Schnittstellen 34 und 35 gesperrt wird, kann eine Dateneingabe über die Schnittstellen 32 und 31 prinzipiell möglich sein, da diesen eine höhere Priorität zugeordnet ist. Wird beispielsweise durch das Smartphone 22 über Schnittstelle 32 versucht eine Dateneingabe zu beginnen, so muss zunächst der Dateneingabe, welcher über Schnittstelle 33 erfolgt, abgebrochen oder unterbrochen werden. Je nach Dateneingabe kann eine gewisse Zeit in Anspruch genommen werden, bis der Prozess erfolgreich unter- oder abgebrochen werden kann. Für manche Dateneingabe kann beispielsweise zusätzlich auch gelten, dass diese grundsätzlich nicht unter- oder abgebrochen werden können. Dies ist zum Beispiel der Fall für Firmware-Aktualisierungen, bei denen ein Abbruch unter anderem auch zu Fehlfunktionen des Beatmungsgeräts führen können.

Während, zu Beginn und/oder auch nach Beendigung der Dateneingabe über Schnittstelle 33 kann eine Meldung oder Information beispielsweise über die Schnittstelle 31 ausgeben werden, welche über die Dateneingabe informiert. Insbesondere bei einer Meldung über die Dateneingabe nach Beendigung der Dateneingabe kann zusätzlich in der Meldung eine Zusammenfassung der durchgeführten Änderung ausgegeben werden.

Für manche Dateneingabe kann zusätzlich eine Bestätigung über die Schnittstelle höchster Priorität oder auch über eine andere Schnittstelle erforderlich sein.

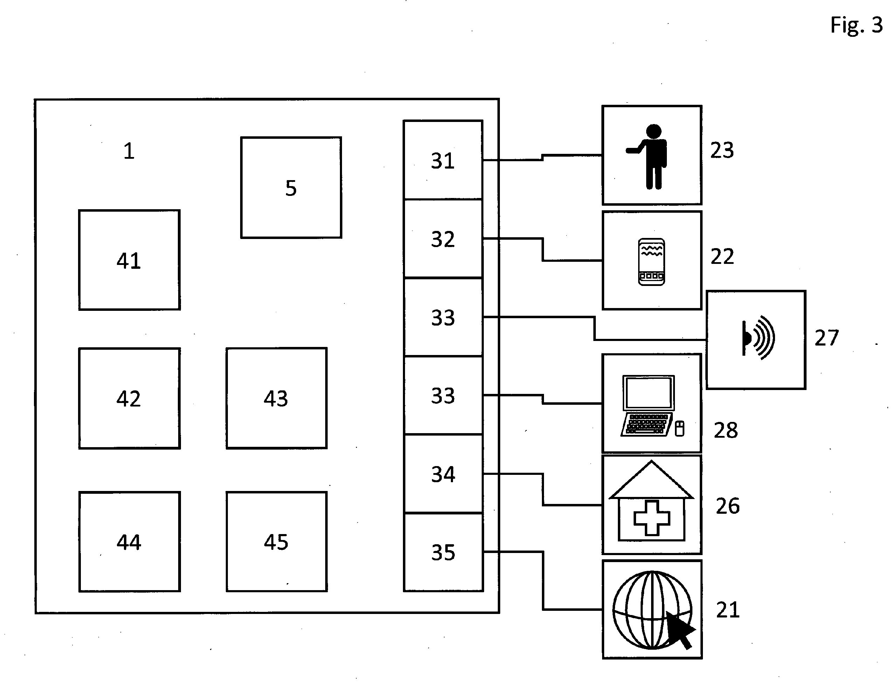

In Figur 3 ist eine beispielhafte Ausführungsform des Beatmungsgerätes 1 dargestellt, welches in weiten Teilen mit dem aus Figur 1 übereinstimmt. Im Unterschied zum Beatmungsgerät 1 aus Figur 1 verfügt das Beatmungsgerät 1 in Figur 3 über zwei Schnittstellen 33. Diese Schnittstellen 33 sind von der gleichen Art und würden zum Beispiel in einer festen Prioritätsliste den gleichen Stellenwert einnehmen, also die gleiche Priorität zugeordnet bekommen. Die Schnittstellen 33 sind beispielsweise so eingerichtet, dass über sie unter anderem ein Sensor 27 und ein Servicegerät 28 mit dem Beatmungsgerät 1 verbunden werden können. Da beide Schnittstellen 33 von der gleichen Art, sind die Schnittstellen 33 also auch austauschbar, das heißt, dass sowohl Sensor 27 als auch Servicegerät 28 mit jeder der beiden Schnittstellen 33 verbunden werden können. Auch wenn die Schnittstellen 33 beide die gleiche Priorität zugeordnet ist, so kann es sein, dass das Servicegerät 28 zeitweise die Gegenstelle mit höchster Priorität sein muss oder auch außerhalb der Prioritätszuordnungen Dateneingaben vornehmen können muss, insofern also auch über der Priorität der Schnittstelle höchster Priorität - in diesem Ausführungsbeispiel Schnittstelle 31 - stehen sollte. Für diesen Fall kann zum einen eine dynamische Priorität eingeführt werden, wonach beispielsweise neben der reinen Schnittstelle auch die verbundene Gegenstelle in die Priorität miteingebunden wird. Nach dieser dynamischen Prioritätsvergabe bekommt beispielsweise eine der Schnittstellen 33 bei Verbindung mit Servicegerät 28 automatisch eine hohe oder die höchste Priorität zugeordnet. Erfolgt nun also eine Dateneingabe über Schnittstelle 33 durch das Servicegerät 28, wird dadurch die Dateneingabe über die anderen Schnittstellen durch die Steuereinheit 5 gesperrt oder zumindest für die von der Dateneingabe vom Servicegerät 28 betroffenen Bestandteile oder Module gesperrt. In manchen Ausführungsformen kann die Dateneingabe aus vom Sensor 27 oder ähnlichen Geräten, wie zum Beispiel Diagnose-Geräten, von der Sperrung grundsätzlich ausgenommen werden. Beispielsweise kann hier über den Typ der Dateneingabe entschieden werden. Sensordaten können dabei beispielsweise anders gehandhabt werden als Dateneingaben zur Konfiguration von Therapiemaßnahmen. Auch die Regelung über verschiedene Dateneingabemodi kann hier verwendet werden. So wird durch den Sensor 26 ein Dateneingabemodus gestartet, welcher nur die Dateneingabe von Sensordaten umfasst. Dieser Dateneingabemodus kann so eingestellt sein, dass nicht Sensordaten allgemein, sondern beispielsweise auch nur bestimmte Sensordaten umfasst werden. So können auch noch weitere Sensoren mit dem Beatmungsgerät verbunden werden, welche andere Sensordaten eingeben und dafür ebenfalls extra Dateneingabemodi starten. Über die Sperrung von Dateneingabetypen oder auch durch Verwendung von Dateneingabemodi kann so auch erreicht werden, dass Dateneingaben über verschiedene Schnittstellen gleichzeitig möglich sind, dadurch, dass aber bereits belegte Dateneingabetypen oder Dateneingabemodi gesperrt werden, können keine Dateneingaben in Konflikt treten. Darüber hinaus ist es beispielsweise auch denkbar, dass bestimmte Dateneingaben dennoch mehrere Dateneingabemodi bzw. -typen sperren. Zum Beispiel kann durch das Einspielen einer Firmware-Aktualisierung über Schnittstelle 33 durch das Servicegerät 28 weitere Dateneingaben zunächst gesperrt werden, um den Erfolg der Aktualisierung nicht zu gefährden. Im Gegenzug kann es beispielsweise auch eingerichtet sein, dass bestimmte Aktionen des Beatmungsgerätes 1, beispielsweise der Beginn einer Therapie, einige Dateneingaben bzw. Dateneingabemodi für alle Schnittstellen sperren. Eine Dateneingabe in Form einer Firmware-Aktualisierung kann so zum Beispiel während der Durchführung einer Therapie verhindert bzw. gesperrt werden. Diese Sperren können beispielsweise fest voreingestellt werden oder auch durch eine Markierung oder ähnliches mit den jeweiligen Dateneingaben mitgesendet werden, sodass beispielsweise die Steuereinheit erkennt, ob eine Dateneinabe abgebrochen werden darf oder welche Dateneingaben parallel erfolgen dürfen.

Anstatt oder auch zusätzlich zu der dynamischen Vergabe von Prioritäten an die Schnittstellen kann auch eine Überbrückung oder eine Umgehung der Prioritäten durch bestimmte Eingabemodi oder Kombinationen von Schnittstellen und Gegenstellen vorgesehen sein. So kann beispielsweise ein Servicegerät 28 bei Verbindung mit Schnittstelle 33 die Prioritäten umgehen oder überbrücken und so auch Dateneingaben tätigen und dabei alle anderen Schnittstellen, unabhängig der (vorgesehenen) Priorität sperren. Formal wird die Schnittstelle 33, welche mit dem Servicegerät 28 verbunden ist, also auf die höchste Priorität gesetzt. Dieses Vorgehen kann dabei auch auf beliebige Kombinationen von Schnittstellen und Gegenstellen übertragen werden.

Auch kann eine der Schnittstellen 31, 32, 33, 34, 35 als eine USB-Schnittstelle oder eine Schnittstelle für SD-Karten ausgebildet sein, welchen eine niedrige Priorität zugeordnet ist. In manchen Fällen können die mit solchen Schnittstellen verbundenen Gegenstellen zum Beispiel Masterkonfigurationen enthalten, welche auf das Beatmungsgerät 1 übertragen werden sollen. Während der Übertragung, also der Dateneingabe über z.B. die SD-Karten- oder USB-Schnittstelle, sollen keine anderen Dateneingaben möglich sein oder zumindest die Dateneingabe über die SD-Karten- oder USB-Schnittstelle die höchste Priorität zugeordnet sein. Dies kann beispielsweise dadurch erreicht werden, dass die Prioritätszuordnung umgangen wird, der SD-Karten- oder USB-Schnittstelle also irregulär die höchste Priorität zugeordnet wird. Als Auslöser für die Umgehung der Prioritätszuordnung sind verschiedene Methoden denkbar. Beispielsweise kann über die entsprechende Gegenstelle, z.B. ein USB-Stick, ein Code übertragen werden, welcher die Umgehung der Prioritätszuordnung startet bzw. die höchste Priorität anfordert. Auch kann eine Bestätigung bzw. Aktion über die Benutzerschnittstelle 31 oder eine andere Schnittstelle dazu führen, dass die Prioritätszuordnung von der SD-Karten- oder USB-Schnittstelle umgangen werden soll bzw. wird. Etwa kann beispielsweise über die Benutzerschnittstelle anzeigt werden, dass ein USB-Stick oder eine SD-Karte mit einer Masterkonfiguration mit dem Beatmungsgerät 1 verbunden wurde und eine Dateneingabe über die SD-Karten- oder USB-Schnittstelle zu starten ist. Wird dies bestätigt, so wird mit der Dateneingabe über die SD-Karten- oder USB-Schnittstelle begonnen und die anderen Schnittstellen werden zumindest teilweise für Dateneingaben gesperrt.

Auch kann die Steuereinheit 5 so eingerichtet sein, dass diese die auf dem Speichermedium (USB Stick, SD-Karte) befindlichen Daten überprüft und anhand des Inhalts entscheidet, ob eine vorübergehende, hohe Priorität für die Schnittstelle gegeben wird beziehungsweise die Prioritätszuordnung umgangen wird.

### Bezugszeichenliste

- 1: Beatmungsgerät
- 5: Steuereinheit
- 21: Internet
- 22: Smartphone
- 23: Anwender
- 24: Computer
- 25: USB Stick
- 26: Gebäudenetzwerk
- 27: Sensor
- 28: Servicegerät
- 31: Schnittstelle
- 32: Schnittstelle
- 33: Schnittstelle
- 34: Schnittstelle
- 35: Schnittstelle
- 41: Modul
- 42: Modul
- 43: Modul
- 44: Modul
- 45: Modul

## Patentansprüche

1. Verfahren zur Verwaltung von Datenaustausch über zumindest drei Schnittstellen (31, 32, 33, 34, 35) eines Beatmungsgerätes (1), wobei zumindest eine Schnittstelle (31, 32, 33, 34, 35) für einen Datenaustausch mit zumindest einer von dem Beatmungsgerät (1) räumlich getrennten Gegenstelle (21, 22, 23) eingerichtet ist, wobei der Datenaustausch eine Dateneingabe und eine Datenausgabe umfasst, wobei eine Dateneingabe über eine der Schnittstellen (31, 32, 33, 34, 35) die Dateneingabe über zumindest eine andere Schnittstelle (31, 32, 33, 34, 35) sperrt, **dadurch gekennzeichnet, dass** den Schnittstellen (31, 32, 33, 34, 35) eine Priorität zugeordnet wird und die Dateneingabe über eine Schnittstelle (31, 32, 33, 34, 35) mit höherer Priorität die Dateneingabe über zumindest eine Schnittstelle (31, 32, 33, 34, 35) niedrigerer Priorität sperrt.

2. Verfahren nach Anspruch 1, **dadurch gekennzeichnet, dass** eine Datenausgabe und/oder Dateneingabe zu jeder Zeit während des laufenden Betriebs des Beatmungsgerätes und bei ausreichenden Systemressourcen und einer sicheren Stromzufuhr über zumindest eine der Schnittstellen (31, 32, 33, 34, 35) möglich ist.

3. Verfahren nach zumindest einem der vorhergehenden Ansprüche, **dadurch gekennzeichnet, dass** die Dateneingabe über zumindest eine Schnittstelle (31, 32, 33, 34, 35) niedrigerer Priorität gesperrt wird, wenn die Dateneingabe über Schnittstellen (31, 32, 33, 34, 35) niedrigerer Priorität mit der Dateneingabe über die Schnittstelle (31, 32, 33, 34, 35) höherer Priorität in Konflikt steht.

4. Verfahren nach zumindest einem der vorhergehenden Ansprüche, **dadurch gekennzeichnet, dass** das Beatmungsgerät (1) zumindest drei verschiedene Schnittstellen (31, 32, 33, 34, 35) umfasst, wobei durch diese zumindest drei Schnittstellen (31, 32, 33, 34, 35) zumindest drei der Gruppen a bis e repräsentiert werden:
a. Drahtlose Schnittstellen für große Entfernungen
b. Drahtlose Schnittstellen für kleine Entfernungen
c. Benutzerschnittstelle
d. Kabelgebundene Schnittstellen
e. Schnittstellen für ein Speichermedium

5. Verfahren nach zumindest einem der vorhergehenden Ansprüche, **dadurch gekennzeichnet, dass** die drahtlosen Schnittstellen (31, 32, 33, 34, 35) des Beatmungsgerätes (1) aktiviert und deaktiviert werden können und eine der Schnittstellen (31, 32, 33, 34, 35) eine Benutzerschnittstelle am Beatmungsgerät (1) ist, wobei der Benutzerschnittstelle die höchste Priorität bezüglich der Dateneingabe zugeordnet wird.

6. Verfahren nach zumindest einem der vorhergehenden Ansprüche, **dadurch gekennzeichnet, dass** über zumindest eine Schnittstelle (31, 32, 33, 34, 35) die Prioritätszuordnung umgangen werden kann, sodass Dateneingaben über diese Schnittstelle den Dateneingaben auch über Schnittstellen (31, 32, 33, 34, 35) der höchsten Priorität vorgezogen werden.

7. Verfahren nach zumindest einem der vorhergehenden Ansprüche, **dadurch gekennzeichnet, dass** zur Dateneingabe ein Dateneingabemodus gestartet wird und das Starten des Dateneingabemodus über eine Schnittstelle (31, 32, 33, 34, 35) das Starten eines Dateneingabemodus über die anderen Schnittstellen (31, 32, 33, 34, 35) sperrt, wobei für verschiedene Dateneingaben jeweils verschiedene Dateneingabemodi gestartet werden, wobei das Starten eines Dateneingabemodus über eine Schnittstelle (31, 32, 33, 34, 35) das Starten des gleichen Dateneingabemodus durch zumindest eine andere Schnittstelle (31, 32, 33, 34, 35) niedrigerer Priorität sperrt und/oder das Starten eines Dateneingabemodus für eine Dateneingabe über andere Schnittstellen (31, 32, 33, 34, 35) gesperrt wird, wenn der zu startende Dateneingabemodus über Schnittstellen (31, 32, 33, 34, 35) niedrigerer Priorität mit dem Dateneingabemodus der Dateneingabe über Schnittstellen (31, 32, 33, 34, 35) höherer Priorität im Konflikt steht.

8. Verfahren nach zumindest einem der vorhergehenden Ansprüche, **dadurch gekennzeichnet, dass** die Dateneingabe über eine Schnittstelle (31, 32, 33, 34, 35) durch den Beginn einer Dateneingabe durch eine Schnittstelle (31, 32, 33, 34, 35), der eine höhere Priorität zugeordnet ist, unterbrochen und/oder abgebrochen wird, wobei bestimmte Dateneingaben unabhängig von der Priorität der Schnittstelle (31, 32, 33, 34, 35) über welche die Dateneingabe erfolgt nicht unterbrochen und/oder abgebrochen werden.

9. Verfahren nach zumindest einem der vorhergehenden Ansprüche, **dadurch gekennzeichnet, dass** die Aktivierung von Funktionen und/oder Arbeitsweisen des Beatmungsgeräts (1) den Datenaustausch über zumindest eine Schnittstelle (31, 32, 33, 34, 35) sperrt und/oder zumindest eine Schnittstelle (31, 32, 33, 34, 35) bis zur Beendigung der Funktion und/oder Arbeitsweise deaktiviert.

10. Verfahren nach zumindest einem der vorhergehenden Ansprüche, **dadurch gekennzeichnet, dass** durch Dateneingaben und/oder Datenausgaben über eine Schnittstelle (31, 32, 33, 34, 35) eine Meldung generiert wird, welche über zumindest eine Schnittstelle (31, 32, 33, 34, 35) höherer Priorität ausgegeben wird und die Meldung nach der Dateneingabe bestehen bleibt und optional über die Schnittstelle (31, 32, 33, 34, 35), über welche die Meldung ausgegeben wird, bestätigt werden muss und die Meldung eine Zusammenfassung über die erfolgte Dateneingabe enthält.

11. Verfahren nach zumindest einem der vorhergehenden Ansprüche, **dadurch gekennzeichnet, dass** den Schnittstellen (31, 32, 33, 34, 35) die Priorität automatisch zugeordnet wird, wobei die Priorität der Schnittstellen (31, 32, 33, 34, 35) am Beatmungsgerät (1) manuell veränderbar ist und die Priorität der Schnittstellen (31, 32, 33, 34, 35) zumindest für den laufenden Betrieb und den nicht-laufenden Betrieb unterschiedlich vergeben wird.

12. Verfahren nach zumindest einem der vorhergehenden Ansprüche, **dadurch gekennzeichnet, dass** das Beatmungsgerät (1) vom laufenden Betrieb in den nicht-laufenden Betrieb übergeht, sobald eine Verbindung über die USB-Schnittstelle hergestellt wird und bei der Herstellung einer Verbindung über die USB-Schnittstelle eine Dateneingabe über alle anderen Schnittstellen (31, 32, 33, 34, 35) gesperrt wird.

13. Verfahren nach zumindest einem der vorhergehenden Ansprüche, **dadurch gekennzeichnet, dass** eine Änderung des Beatmungsgeräts (1) vom laufenden in den nicht-laufenden Betrieb oder umgekehrt zumindest zeitweise über alle Schnittstellen (31, 32, 33, 34, 35) gesperrt wird, nachdem eine solche Änderung vorgenommen wurde.

14. Verfahren nach zumindest einem der vorhergehenden Ansprüche, **dadurch gekennzeichnet, dass** im Wesentlichen alle drahtlosen Verbindungen über entsprechende Schnittstellen (31, 32, 33, 34, 35) des Beatmungsgerätes (1) abgebrochen werden und die Schnittstellen (31, 32, 33, 34, 35) für drahtlose Verbindungen deaktiviert werden, wenn das Beatmungsgerät (1) in den laufenden Betrieb übergeht und/oder übergehen soll, wobei das Beatmungsgerät (1) nicht in den laufenden Betrieb übergeht bis alle drahtlosen Verbindungen unterbrochen und die Schnittstellen (31, 32, 33, 34, 35) für drahtlose Verbindungen deaktiviert sind.

15. Beatmungsgerät (1) umfassend zumindest drei Schnittstellen (31, 32, 33, 34, 35) für einen Datenaustausch, wobei zumindest eine Schnittstelle (31, 32, 33, 34, 35) für einen Datenaustausch mit zumindest einer von dem Beatmungsgerät (1) räumlich getrennten Gegenstelle (21, 22, 23, 24, 25) eingerichtet ist, wobei der Datenaustausch eine Dateneingabe und eine Datenausgabe umfasst, wobei das Beatmungsgerät derart eingerichtet ist, dass eine Dateneingabe über eine der Schnittstellen (31, 32, 33, 34, 35) die Dateneingabe über zumindest eine andere Schnittstelle (31, 32, 33, 34, 35) sperrt, **dadurch gekennzeichnet, dass** den Schnittstellen (31, 32, 33, 34, 35) eine Priorität zugeordnet ist und das Beatmungsgerät derart eingerichtet ist, dass die Dateneingabe über eine Schnittstelle (31, 32, 33, 34, 35) mit höherer Priorität die Dateneingabe über zumindest eine Schnittstelle (31, 32, 33, 34, 35) niedrigerer Priorität sperrt.

16. Beatmungsgerät (1) nach Anspruch 15, **dadurch gekennzeichnet, dass** das Beatmungsgerät derart eingerichtet ist, dass eine Datenausgabe zu jeder Zeit während des laufenden Betriebs des Beatmungsgerätes und bei ausreichenden Systemressourcen und einer sicheren Stromzufuhr über zumindest eine der Schnittstellen (31, 32, 33, 34, 35) möglich ist und den Schnittstellen (31, 32, 33, 34, 35) eine Priorität zugeordnet wird und die Dateneingabe über eine Schnittstelle (31, 32, 33, 34, 35) mit höherer Priorität die Dateneingabe über zumindest eine Schnittstelle (31, 32, 33, 34, 35) niedrigerer Priorität sperrt.

17. Beatmungsgerät (1) nach zumindest einem der Ansprüche 15 oder 16, **dadurch gekennzeichnet, dass** das Beatmungsgerät (1) zumindest drei verschiedene Schnittstellen (31, 32, 33, 34, 35) umfasst, wobei durch diese zumindest drei Schnittstellen (31, 32, 33, 34, 35) zumindest drei der Gruppen a bis e repräsentiert werden:
a. Drahtlose Schnittstellen für große Entfernungen
b. Drahtlose Schnittstellen für kleine Entfernungen
c. Benutzerschnittstelle
d. Kabelgebundene Schnittstellen
e. Schnittstellen für ein Speichermedium

## Claims

1. A method for managing data exchange via at least three interfaces (31, 32, 33, 34, 35) of a ventilator (1), wherein at least one interface (31, 32, 33, 34, 35) is configured for a data exchange with at least one counterpart station (21, 22, 23) that is spatially separate from the ventilator (1), the data exchange comprising a data input and a data output, and a data input via one of the interfaces (31, 32, 33, 34, 35) blocking the data input via at least one other interface (31, 32, 33, 34, 35), **characterized in that** a priority is assigned to the interfaces (31, 32, 33, 34, 35) and the data input via an interface (31, 32, 33, 34, 35) with higher priority blocks the data input via at least one interface (31, 32, 33, 34, 35) of lower priority.

2. The method of claim 1, **characterized in that** a data output and/or data input are/is possible at any time during the ongoing operation of the ventilator and given sufficient system resources and a reliable power supply via at least one of the interfaces (31, 32, 33, 34, 35).

3. The method according to at least one of the preceding claims, **characterized in that** the data input via at least one interface (31, 32, 33, 34, 35) of lower priority is blocked if the data input via interfaces (31, 32, 33, 34, 35) of lower priority conflicts with the data input via the interface (31, 32, 33, 34, 35) of higher priority.

4. The method according to at least one of the preceding claims, **characterized in that** the ventilator (1) comprises at least three different interfaces (31, 32, 33, 34, 35), wherein at least three of groups a to e are represented by said at least three interfaces (31, 32, 33, 34, 35):
a. Wireless interfaces for large distances
b. Wireless interfaces for small distances
c. User interface
d. Wired interfaces
e. Interfaces for a storage medium

5. The method according to at least one of the preceding claims, **characterized in that** the wireless interfaces (31, 32, 33, 34, 35) of the ventilator (1) can be activated and deactivated and one of the interfaces (31, 32, 33, 34, 35) is a user interface at the ventilator (1), the user interface being assigned the highest priority with regard to the data input.

6. The method according to at least one of the preceding claims, **characterized in that** the priority assignment can be circumvented via at least one interface (31, 32, 33, 34, 35), such that data inputs via this interface are given preference over the data inputs even via interfaces (31, 32, 33, 34, 35) of the highest priority.

7. The method according to at least one of the preceding claims, **characterized in that** for data input purposes a data input mode is started and starting of the data input mode via one interface (31, 32, 33, 34, 35) blocks starting of a data input mode via the other interfaces (31, 32, 33, 34, 35), wherein different data input modes are in each case started for different data inputs, wherein starting of a data input mode via one interface (31, 32, 33, 34, 35) blocks starting of the same data input mode by at least one other interface (31, 32, 33, 34, 35) of lower priority and/or starting of a data input mode for a data input via other interfaces (31, 32, 33, 34, 35) is blocked if the data input mode to be started via interfaces (31, 32, 33, 34, 35) of lower priority conflicts with the data input mode of the data input via interfaces (31, 32, 33, 34, 35) of higher priority.

8. The method according to at least one of the preceding claims, **characterized in that** the data input via an interface (31, 32, 33, 34, 35) is interrupted and/or terminated by the beginning of a data input by an interface (31, 32, 33, 34, 35) to which a higher priority is assigned, specific data inputs being not interrupted and/or terminated regardless of the priority of the interface (31, 32, 33, 34, 35) via which the data input is effected.

9. The method according to at least one of the preceding claims, **characterized in that** the activation of functions and/or modes of operation of the ventilator (1) blocks the data exchange via at least one interface (31, 32, 33, 34, 35) and/or deactivates at least one interface (31, 32, 33, 34, 35) until the end of the function and/or mode of operation.

10. The method according to at least one of the preceding claims, **characterized in that** a message is generated by data inputs and/or data outputs via an interface (31, 32, 33, 34, 35), the message being output via at least one interface (31, 32, 33, 34, 35) of higher priority, and the message persists after the data input and must optionally be confirmed via the interface (31, 32, 33, 34, 35) via which the message is output, and the message contains a summary of the data input effected.

11. The method according to at least one of the preceding claims, **characterized in that** the priority is automatically assigned to the interfaces (31, 32, 33, 34, 35), wherein the priority of the interfaces (31, 32, 33, 34, 35) can be varied manually at the ventilator (1) and the priority of the interfaces (31, 32, 33, 34, 35) is allocated differently at least for ongoing operation and non-ongoing operation.

12. The method according to at least one of the preceding claims, **characterized in that** the ventilator (1) transitions from ongoing operation to non-ongoing operation as soon as a connection via the USB interface is established, and a data input via all other interfaces (31, 32, 33, 34, 35) is blocked when a connection via the USB interface is established.

13. The method according to at least one of the preceding claims, **characterized in that** a change in the ventilator (1) from ongoing to non-ongoing operation, or vice versa, is blocked at least at times via all interfaces (31, 32, 33, 34, 35) after such a change has been made.

14. The method according to at least one of the preceding claims, **characterized in that** substantially all wireless connections via corresponding interfaces (31, 32, 33, 34, 35) of the ventilator (1) are terminated and the interfaces (31, 32, 33, 34, 35) for wireless connections are deactivated if the ventilator (1) transitions and/ or is intended to transition to ongoing operation, wherein the ventilator (1) does not transition to ongoing operation until all wireless connections are interrupted and the interfaces (31, 32, 33, 34, 35) for wireless connections are deactivated.

15. A ventilator (1), comprising at least three interfaces (31, 32, 33, 34, 35) for a data exchange, at least one interface (31, 32, 33, 34, 35) being configured for a data exchange with at least one counterpart station (21, 22, 23, 24, 25) that is spatially separate from the ventilator (1), the data exchange comprising a data input and a data output, wherein the ventilator is configured such that a data input via one of the interfaces (31, 32, 33, 34, 35) blocks the data input via at least one other interface (31, 32, 33, 34, 35), **characterized in that** a priority is assigned to the interfaces (31, 32, 33, 34, 35) and the ventilator is configured such that the data input via an interface (31, 32, 33, 34, 35) with higher priority blocks the data input via at least one interface (31, 32, 33, 34, 35) of lower priority.

16. The ventilator (1) of claim 15, **characterized in that** the ventilator is configured such that a data output is possible at any time during ongoing operation of the ventilator and given sufficient system resources and a reliable power supply via at least one of the interfaces (31, 32, 33, 34, 35), and a priority is assigned to the interfaces (31, 32, 33, 34, 35) and the data input via an interface (31, 32, 33, 34, 35) with higher priority blocks the data input via at least one interface (31, 32, 33, 34, 35) of lower priority

17. The ventilator (1) according to at least one of claims 15 or 16, **characterized in that** the ventilator (1) comprises at least three different interfaces (31, 32, 33, 34, 35), wherein at least three of groups a to e are represented by said at least three interfaces (31, 32, 33, 34, 35):
a. Wireless interfaces for large distances
b. Wireless interfaces for small distances
c. User interface
d. Wired interfaces
e. Interfaces for a storage medium

## Revendications

1. Procédé pour la gestion de l'échange de données par au moins trois interfaces (31, 32, 33, 34, 35) d'un appareil respiratoire (1), au moins une interface (31, 32, 33, 34, 35) étant agencée pour un échange de données avec au moins un correspondant (21, 22, 23) séparé spatialement de l'appareil respiratoire (1), l'échange de données comprenant une entrée de données et une sortie de données, une entrée de données par une des interfaces (31, 32, 33, 34, 35) bloquant l'entrée de données par au moins une autre interface (31, 32, 33, 34, 35), **caractérisé en ce qu'**une priorité est attribuée aux interfaces (31, 32, 33, 34, 35) et **en ce que** l'entrée de données par une interface (31, 32, 33, 34, 35) avec priorité supérieure bloque l'entrée de données par au moins une interface (31, 32, 33, 34, 35) de priorité inférieure.

2. Procédé selon la revendication 1, **caractérisé en ce qu'**une sortie de données et/ou une entrée de données est possible à tout moment par au moins une des interfaces (31, 32, 33, 34, 35) pendant le fonctionnement en cours de l'appareil respiratoire et avec des ressources de système suffisantes et une alimentation électrique sûre.

3. Procédé selon au moins l'une des revendications précédentes, **caractérisé en ce que** l'entrée de données par au moins une interface (31, 32, 33, 34, 35) de priorité inférieure est bloquée lorsque l'entrée de données par des interfaces (31, 32, 33, 34, 35) de priorité inférieure est en conflit avec l'entrée de données par l'interface (31, 32, 33, 34, 35) de priorité supérieure.

4. Procédé selon au moins l'une des revendications précédentes, **caractérisé en ce que** l'appareil respiratoire (1) comprend au moins trois interfaces (31, 32, 33, 34, 35) différentes, au moins trois des groupes a à e étant représentés par ces au moins trois interfaces (31, 32, 33, 34, 35) :
a. Interfaces sans fil pour grandes distances
b. Interfaces sans fil pour distances courtes
c. Interface utilisateur
d. Interfaces reliées par câble
e. Interfaces pour un support de stockage

5. Procédé selon au moins l'une des revendications précédentes, **caractérisé en ce que** les interfaces sans fil (31, 32, 33, 34, 35) de l'appareil respiratoire (1) peuvent être activées et désactivées et **en ce qu'**une des interfaces (31, 32, 33, 34, 35) est une interface utilisateur sur l'appareil respiratoire (1), la priorité la plus élevée relativement à l'entrée de données étant attribuée à l'interface utilisateur.

6. Procédé selon au moins l'une des revendications précédentes, **caractérisé en ce que** l'attribution de priorité peut être contournée par au moins une interface (31, 32, 33, 34, 35) de telle sorte que des entrées de données par cette interface sont préférées aux entrées de données également par des interfaces (31, 32, 33, 34, 35) de la priorité la plus élevée.

7. Procédé selon au moins l'une des revendications précédentes, **caractérisé en ce qu'**un mode d'entrée de données est démarré pour l'entrée de données, et **en ce que** le démarrage du mode d'entrée de données par une interface (31, 32, 33, 34, 35) bloque le démarrage d'un mode d'entrée de données par les autres interfaces (31, 32, 33, 34, 35), différents modes d'entrée de données étant respectivement démarrés pour différentes entrées de données, le démarrage d'un mode d'entrée de données par une interface (31, 32, 33, 34, 35) bloquant le démarrage du même mode d'entrée de données par au moins une autre interface (31, 32, 33, 34, 35) de priorité inférieure, et/ou le démarrage d'un mode d'entrée de données pour une entrée de données par d'autres interfaces (31, 32, 33, 34, 35) étant bloqué lorsque le mode d'entrée de données devant être démarré par des interfaces (31, 32, 33, 34, 35) de priorité inférieure est en conflit avec le mode d'entrée de données de l'entrée de données par des interfaces (31, 32, 33, 34, 35) de priorité supérieure.

8. Procédé selon au moins l'une des revendications précédentes, **caractérisé en ce que** l'entrée de données par une interface (31, 32, 33, 34, 35) est interrompue et/ou annulée par le commencement d'une entrée de données par une interface (31, 32, 33, 34, 35) à laquelle une priorité supérieure est attribuée, des entrées de données spécifiques n'étant pas interrompues et/ou annulées indépendamment de la priorité de l'interface (31, 32, 33, 34, 35) par laquelle l'entrée de données est effectuée.

9. Procédé selon au moins l'une des revendications précédentes, **caractérisé en ce que** l'activation de fonctions et/ou de modes de travail de l'appareil respiratoire (1) bloque l'échange de données par au moins une interface (31, 32, 33, 34, 35) et/ou désactive au moins une interface (31, 32, 33, 34, 35) jusqu'à l'achèvement de la fonction et/ou du mode de travail.

10. Procédé selon au moins l'une des revendications précédentes, **caractérisé en ce qu'**un message est généré par des entrée de données et/ou des sorties de données par une interface (31, 32, 33, 34, 35), lequel message est sorti par au moins une interface (31, 32, 33, 34, 35) de priorité supérieure, et **en ce que** le message est maintenu après l'entrée de données et, optionnellement, doit être confirmé par l'interface (31, 32, 33, 34, 35) par laquelle le message est sorti, et **en ce que** le message contient un résumé sur l'entrée de données effectuée.

11. Procédé selon au moins l'une des revendications précédentes, **caractérisé en ce que** la priorité est attribuée automatiquement aux interfaces (31, 32, 33, 34, 35), la priorité des interfaces (31, 32, 33, 34, 35) pouvant être modifiée manuellement sur l'appareil respiratoire (1), et la priorité des interfaces (31, 32, 33, 34, 35) étant attribuée différemment au moins pour le fonctionnement en cours et le fonctionnement non en cours.

12. Procédé selon au moins l'une des revendications précédentes, **caractérisé en ce que** l'appareil respiratoire (1) passe du fonctionnement en cours au fonctionnement non en cours dès qu'une liaison est établie par l'interface USB et qu'une entrée de données par toutes les autres interfaces (31, 32, 33, 34, 35) est bloquée lors de l'établissement d'une liaison par l'interface USB.

13. Procédé selon au moins l'une des revendications précédentes, **caractérisé en ce qu'**un passage de l'appareil respiratoire (1) du fonctionnement en cours au fonctionnement non en cours ou inversement est bloqué au moins temporairement par toutes les interfaces (31, 32, 33, 34, 35) après qu'un tel passage a été effectué.

14. Procédé selon au moins l'une des revendications précédentes, **caractérisé en ce que** toutes les liaisons sans fil par des interfaces correspondantes (31, 32, 33, 34, 35) de l'appareil respiratoire (1) sont essentiellement annulées, et **en ce que** les interfaces (31, 32, 33, 34, 35) pour les liaisons sans fil sont désactivées lorsque l'appareil respiratoire (1) passe et/ou doit passer en fonctionnement en cours, l'appareil respiratoire (1) ne passant pas en fonctionnement en cours avant que toutes les liaisons sans fil soient interrompues et que les interfaces (31, 32, 33, 34, 35) pour les liaisons sans fil soient désactivées.

15. Appareil respiratoire (1) comprenant au moins trois interfaces (31, 32, 33, 34, 35) pour un échange de données, au moins une interface (31, 32, 33, 34, 35) étant agencée pour un échange de données avec au moins un correspondant (21, 22, 23, 24, 25) séparé spatialement de l'appareil respiratoire (1), l'échange de données comprenant une entrée de données et une sortie de données, l'appareil respiratoire étant agencé de telle sorte qu'une entrée de données par une des interfaces (31, 32, 33, 34, 35) bloque l'entrée de données par au moins une autre interface (31, 32, 33, 34, 35), **caractérisé en ce qu'**une priorité est attribuée aux interfaces (31, 32, 33, 34, 35) et l'appareil respiratoire étant agencé de telle sorte que l'entrée de données par une interface (31, 32, 33, 34, 35) de priorité supérieure bloque l'entrée de données par au moins une interface (31, 32, 33, 34, 35) de priorité inférieure.

16. Appareil respiratoire (1) selon la revendication 15, **caractérisé en ce que** l'appareil respiratoire est agencé de telle sorte qu'une sortie de données est possible à tout moment par au moins une des interfaces (31, 32, 33, 34, 35) pendant le fonctionnement en cours de l'appareil respiratoire et avec des ressources de système suffisantes et une alimentation électrique sûre, et **en ce qu'**une priorité est attribuée aux interfaces (31, 32, 33, 34, 35), et **en ce que** l'entrée de données par une interface (31, 32, 33, 34, 35) avec priorité supérieure bloque l'entrée de données par au moins une interface (31, 32, 33, 34, 35) de priorité inférieure.

17. Appareil respiratoire (1) selon au moins l'une des revendications 15 ou 16, **caractérisé en ce que** l'appareil respiratoire (1) comprend au moins trois interfaces différentes (31, 32, 33, 34, 35), au moins trois des groupes a à e étant représentés par ces au moins trois interfaces (31, 32, 33, 34, 35) :
a. Interfaces sans fil pour grandes distances
b. Interfaces sans fil pour distances courtes
c. Interface utilisateur
d. Interfaces reliées par câble
e. Interfaces pour un support de stockage
